(19) Europäisches Patentamt
European Patent Office
Office européen des brevets

(11) **EP 2 250 987 A2**

(12) **EUROPEAN PATENT APPLICATION**

(43) Date of publication:
**17.11.2010 Bulletin 2010/46**

(51) Int Cl.:
*A61G 7/057* (2006.01)  *A47C 27/08* (2006.01)
*A47C 27/12* (2006.01)  *A47C 27/22* (2006.01)
*A47C 27/14* (2006.01)

(21) Application number: **10172976.2**

(22) Date of filing: **02.05.2005**

(84) Designated Contracting States:
**AT BE BG CH CY CZ DE DK EE ES FI FR GB GR HU IE IS IT LI LT LU MC NL PL PT RO SE SI SK TR**

(30) Priority: **30.04.2004 US 567215 P**

(62) Document number(s) of the earlier application(s) in accordance with Art. 76 EPC:
**05742344.4 / 1 740 143**

(71) Applicant: **Hill-Rom Services, Inc.**
**Wilmington, DE 19801 (US)**

(72) Inventors:
• **Bobey, John, A**
  **Daniel Island, 29492-7548 (US)**
• **Branson, Gregory, W**
  **Batesville, IN 47006 (US)**
• **Clarke, Colin**
  **Victoria British Columbia V8R 6A3 (CA)**
• **Hopkins, Rachel**
  **Lawrenceburg, IN 47025 (US)**
• **Jacques, William, L., III.**
  **Mt. Pleasant, SC 29466 (US)**
• **JanoffNOFF, Karen**
  **Mt. Pleasant, SC 29464 (US)**
• **Lokhorst, David, M**
  **Victoria British Columbia V8Z 5K1 (CA)**
• **Meyer, Eric, R**
  **Greensburg, IN 47240 (US)**

• **Mueller, Jonathan, H**
  **Mount Pleasant, SC 29466 (US)**
• **O'Neal, Todd, P**
  **Fairfield, OH 45014 (US)**
• **Petrosenko, Robert**
  **Daniel Island, SC 29492 (US)**
• **Schulte, Stephen R**
  **Harrison, OH 45030 (US)**
• **Skinner, Andrew**
  **Batesville, IN 47007 (US)**
• **Sleva, Michael Z**
  **Decatus, GA 30030 (US)**
• **Soltani, Sohrab**
  **Charleston, SC 29403 (US)**
• **Stacy, Richard, B**
  **Daniel Island, SC 29492 (US)**
• **Stevens, Daniel, K**
  **Summerville, SC 29483 (US)**
• **Yermaneni, Mayur**
  **Shrewsbury, MA 01545 (US)**

(74) Representative: **Findlay, Alice Rosemary**
**Reddie & Grose**
**16 Theobalds Road**
**London**
**WC1X 8PL (GB)**

Remarks:
This application was filed on 16.08.2010 as a divisional application to the application mentioned under INID code 62.

(54) **Patient support with 3-D fiber material**

(57)     This disclosure describes certain exemplary embodiments of a patient support (10) having a plurality of vertically-oriented on substantially can-shaped inflatable bladders (50). In one embodiment, the patient support includes a plurality of pressure sensors (136) positioned underneath the bladders. In another embodiment, the patient support includes a support layer (20) positioned above the vertical bladders. In still another embodiment, the patient support includes one or more filler portions (80, 84, 86, 90) that are selectable so that the patient support may conform to bed frames, having different deck configurations.

In certain embodiments, a patient support having real time pressure control is provided. The patient support includes a plurality of sensors heated beneath a bladder including a plurality of upright cylindrical elements. The pressure within the bladder is controlled based on the pressure or force sensed by the plurality of sensors.

In certain embodiments, a lack of patient movement monitor and method is provided. The monitor and method patient support includes a plurality of sensors located beneath a patient support to determine movement of a patient. Art alarm is activated when patient movement over time is determined to be lacking.

The present invention also includes a pressure relief patient support (1010) for use in combination with a bed

frame. The pressure relief support surface includes a plurality of layers of a three-dimensional fiber material (1302) positioned above a plurality of vertical air cells (50).

FIG. 1

**Description**

Background of the Disclosure

The present disclosure relates to a device for supporting a patient, such as a mattress. In particular, the present disclosure relates to patient supports appropriate for use in hospitals, acute care facilities, and other patient care environments, Certain embodiments disclosed herein relate to pressure relief support surfaces, or patient support surfaces that are configured to accommodate and operate witch a variety of sizes and styles of beds, bed frames, and patient types.

Summary of the Disclosure

In one illustrated embodiment of the present invention, a patient support is provided that has a cover defining an interior region. A base is positioned in the interior region. Inflatable bladders extend upwardly from the base along a vertical axis. The vertical axis is substantially perpendicular to the base.

Pressure sensors may be positioned underneath the base. The pressure sensors may be arranged so that each sensor is aligned with at least one of the vertical bladders. The pressure sensors may be enclosed within an enclosure. The enclosure may be located in the interior region of the cover.

The pressure sensors may include one or more light transmitters or conductors or optical fibers. The pressure sensors may operate to measure pressure applied to one or more of the bladders. One or more of the pressure sensors may evaluate changes in intensity of light energy diffused within the sensor.

One or more pressure transducers may be coupled to the inflatable bladders. The pressure transducers may operate to measure internal pressure of fluid within the bladders.

A support layer may be positioned above the inflatable bladders. The support layer may have at least one support characteristic that is different from a support characteristic of the inflatable bladders. The support layer may include a breathable or air-permeable material. The support layer may include resilient portions, The support layer may include projections and depressions. The support layer may be enclosed within an enclosure. The enclosure may be located in the interior region of the cover.

The inflatable bladders may be substantially can-shaped or cylindrical, One or more of the inflatable bladders may include a beveled portion located between a top portion and a vertical portion of the bladder.

The patient support may include one or more removable filler portions, The filler portions may be selected to conform the patient support to bed frames having one or more deck configurations, including flat deck and step or recessed deck configurations.

In certain embodiments, the present invention provides an apparatus and method for minimizing the interface pressure between a support surface and a person or patient on the surface.

In the illustrated embodiment of the present invention, a pressure adjustable mattress system. The mattress system includes a plurality of air bladders, a plurality of force sensors, each of the plurality of pressure sensors subtending at least one of the plurality of air bladders to sense a force transmitted through the subtended air bladder, and a plurality of outputs, to transmit a signals representative of a sensed force, each of the plurality of outputs coupled to at least one of the plurality of force sensors.

According to another aspect of the present invention there is provides a method for adjusting the pressure of a pressure adjustable mattress to a pressure value, the mattress including a plurality of force sensors wherein each of the plurality of force sensors generates a force signal representative of a sensed force transmitted through the pressure adjustable mattress. The method includes the steps of ordering the force signals in a predetermined order, calculating an indicator signal as a function of the ordered force signals, comparing the indicator signal to the predetermined value to generate a correction signal, and adjusting the pressure of the pressure adjustable mattress based on the correction signal.

Also there is provided in a pressure adjustable support, including a bladder to support a patient and having a pressure therein, a sensor subtending the bladder to sense a force transmitted through the bladder and generating a signal responsive to the sensed force a method. The method includes the steps of detecting a position of the patient on the bladder with the sensor, detecting movement of the patient on the bladder with the sensor, and adjusting the pressure within the bladder responsive to the detected position and the detected movement of the patient.

With respect to another aspect of the present invention, there is provided in a pressure adjustable support, including a bladder assembly having a plurality of vertical bladders, to support a patient, a plurality of sensors, each of the plurality of sensors subtending at least one of the vertical bladders to sense a force transmitted through the vertical bladders, a method. The method includes the steps of detecting a position of the patient on the plurality of vertical bladders with the plurality off sensors, detecting movement of the patient on the plurality of vertical bladders with the plurality of sensors, and adjusting the pressure within the bladder responsive to the detected position and the detected movement of the patient.

In accordance with one aspect of the present invention, a method is provided for a pressure adjustable support, including a bladder assembly having a plurality of bladders to support a patient, a plurality of sensors, each of the plurality of sensors subtending at least one of the vertical bladders to sense a force transmitted through the bladders. The method includes the steps of: setting a time period for determining an activity level, setting a threshold level with respect to the sensed force, sampling the forces sensed by each of the plurality of sensors transmitted through the bladders; and generating a signal as a function of the set time period, the set threshold level, and the sampled forces.

In another aspect of the present invention there is provided a motion monitor device for monitoring the motion of a patient lying on a hospital bed, inducing a mattress. The motion monitor includes a plurality of sensors subtending the mattress, and a user interface device, operatively coupled to the plurality of sensors, the user interface device including a screen to display motion information and an input device to input motion parameters to determine patient motion.

In one illustrated embodiment, a patient support is provided that has a cover defining an interior region. The cover includes a top surface and a bottom surface. First and second layers of a three-dimensional material and a plurality of vertical can bladders are positioned in the interior region. The plurality of vertical can bladders is positioned below the second layer. The three-dimensional material comprised a network of thermoplastic fibers. The network comprises a plurality of spaced-apart dome-shaped projections. The first layer is positioned with the dome-shaped projections projecting upwardly toward the top surface of the cover. The second layer is positioned below the first layer, The dome-shaped projections of the second layer project downwardly away from the first layer toward the bottom surface of the cover.

In another embodiment, a patient support is provided that has an outer cover defining an interior region. A support layer and a plurality of vertical can bladders are positioned in the interior region. The plurality of vertical can bladders positioned below the support layer. The support layer includes a support cover, an upper section, and a lower section. The upper and lower sections are formed from a three-dimensional material comprising a network of thermoplastic fibers.

In another embodiment, a patient support is provided that has a cover defining an interior region. A body and a top layer are positioned in the interior region. The body includes a plurality of inflatable zones, each zone including a plurality of vertical can bladders. The top layer is positioned above the body in the interior region. The top layer includes at least one layer of an air-permeable three-dimensional material. The three-dimensional material comprises a network of thermoplastic fibers three-dimensional material.

In yet another embodiment, a patient support is provided that has a cover defining an interior region. A first layer and a second layer are located in the interior region. The second layer is positioned below the first layer. The first layer includes an upper section and a lower section, Each of the upper and lower sections includes at least one layer of an air-permeable three-dimensional material. The three-dimensional material comprises a network of thermoplastic fibers. The second layer includes head, seat, and foot sections. At least one of the head, seat, and foot sections include vertical inflatable bladders.

Additional features and advantages of the invention will become apparent to those skilled in the art upon consideration of the following detailed description of illustrated embodiments exemplifying the best mode of carrying out the invention as presently perceived.

Brief Description of the Drawings

Aspects of the present invention are more particularly described below with reference to the following figures, which illustrate exemplary embodiments of the present invention:

Fig. 1 is a perspective view of a patient support positioned on an exemplary hospital bed, with a portion of the patient support being cut away to show interior components of the patient support;

Fig. 2 is a perspective view of a patient support, with a portion being cut away to show interior components of the patient support;

Fig. 3 is an exploded view of components of the illustrated embodiment of a patient support;

Fig. 4 is a schematic view of an exemplary three-dimensional support material;

Fig. 5 is a side view of selected components of the illustrated embodiment of a patient support;

Fig. 6 is a top view of components of a patient support also shown in Fig. 5;

Fig. 7 is a perspective view of a first bladder assembly for a patient support;

Fig. 8 is a side view of the bladder assembly of Fig. 7;

Fig. 9 is a top schematic view of the bladder assembly of Fig. 7;

Fig. 10 is a close-up schematic view of Area A of Fig. 9;

Fig. 11 is a close-up schematic view of Area B of Fig. 9;

Fig. 12A is a top view of a portion of a bladder assembly including a vertical bladder and an inlet tube;

Fig. 12B is a cross-sectional view taken along line B-B of Fig. 12A;

Fig. 12C is a cross-sectional view taken along line C-C of Fig. 9;

Fig. 13 is a cross-sectional view of a portion of a and vertical bladder;

Fig. 14 is a perspective view of a second bladder assembly for a patient support;

Fig. 15 is a top schematic of the bladder of Fig. 14;

Fig. 16 is a perspective of a third bladder assembly for a patient support;

Fig. 17 is another perspective view of the bladder of Fig. 16;

Fig. 18 a side view of the bladder assembly of Fig. 16;

Fig. 19 is a top schematic of the bladder assembly of Fig. 16;

Fig. 20 is a top view of exemplary sensor pads;

Fig. 21 is a perspective of an exemplary sensor pad showing interior components thereof;

Fig. 22 is a perspective view of an exemplary bolster assembly;

Fig. 23 is a schematic view of air zones of the illustrated patient support and associated air supply system;

Figs. 24A and 24B are schematic diagrams of portions of a control system for the illustrated patient support:

Fig. 25 is an exploded of an exemplary pneumatic assembly;

Fig. 26 is a perspective of the pneumatic assembly of Fig. 25;

Fig. 27 illustrates a first and second sensor pad including a sequence of reading data from the of the sensor pad;

Fig. 28 illustrates a functional block diagram illustrating the head zone and seat zone sensors and other system components coupled to a communication network;

Fig. 29 illustrates a block diagram for a control system of the present invention including an algorithm control unit;

Fig. 30 is a flow diagram illustrating one embodiment of the present invention to determine patient movement the patient movement monitor;

Fig. 31 is a screen display of a motion monitor user interface screen when the motion monitor is in the off condition;

Fig. 32 is a screen display of the motion monitor user interface screen when the motion monitor is set to a time period of two hours;

Fig. 33 is a screen of the motion monitor user interface screen when the motion monitor is set to a time of 30 minutes and the minimum motion level is set to a level of 4 out of 5;

Fig. 34 is a screen display of the motion monitor user interface screen when the motion monitor is set to a time period of 30 minutes and the minimum motion level is set to the highest setting;

Fig. 35 is a screen display of the motion monitor user interface screen when the history button is selected to display a default history view the past 24 hours of monitored motion;

Fig. 36 is another embodiment of the screen display of the motion monitor user interface screen when the motion monitor is in the off condition;

Fig. 37 is the screen display of Fig. 14 including the motion monitor alert set to 2 hours;

Fig. 38 is the screen display of Fig. 14, including the motion monitor alert set to set to 2 hours with motion considered to be immobile during the time period;

Fig. 39 is a screen of the motion monitor user interface screen showing an alert notification if the motion level does not rise above the minimum motion level set in the motion monitor;

Fig. 40 illustrates a block diagram for a pressure optimization control system of the present invention;

Fig. 41 illustrates a flow chart illustrating a method of determining a pressure for the patient support of the present invention ;

Fig. 42 illustrates a block diagram of algorithms of the present invention;

Fig. 43 illustrates a flowchart of optimizing pressure in the present invention

Fig. 44 illustrates a state machine diagram for a control system of the present invention;

Fig. 45 illustrates a state machine diagram for a pressure relief control system of the present invention;

Figs. 46a-46f illustrate side views of various configurations of a three-dimensional material;

Fig. 46g is a side view of one embodiment of a three-dimensional spacer material;

Fig. 47 illustrates another configuration of three-dimensional material including two different embodiments of three-dimensional material;

Fig. 48 illustrates a perspective of one embodiment of a support surface including three-dimensional material and a foam base, with a portion of the cover cut away;

Fig. 49 illustrates a perspective view of a second embodiment of a support surface including three-dimensional material and a foam base, with a portion of the cover cut away;

Fig. 50 is top view of another embodiment of a support surface including layers of three-dimensional material, with a portion of the cover cut-a-way;

Fig. 51 is cross section of Fig. 50 along 9-9 showing the interior of the support surface;

Fig. 52 is cross of Fig. 50 along 10-10 showing the interior of the support surface; and

Figs. 53a-53b illustrate side views of various configurations of a three-dimensional material similar to those in Fig. 50.

Detailed Description of Illustrated Embodiments

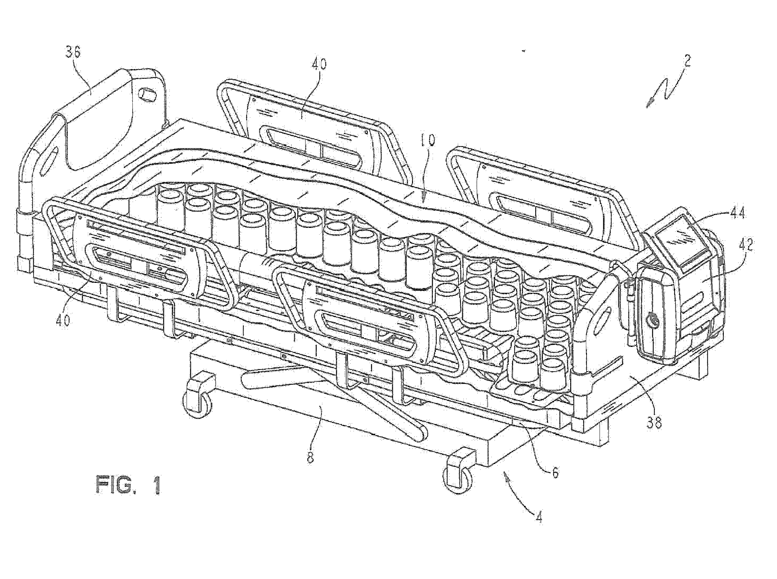

Fig. 1 shows an embodiment of a patient support or mattress 10 in accordance with the present invention. Patient support 10 is positioned on an exemplary bed 2. Bed 2, as illustrated, is a hospital bed including a frame 4, a headboard 36, a footboard 38, and a plurality of siderails 40.

Frame 4 of the exemplary bed 2 generally includes a deck 6 supported by a base 8. Deck 6 includes one or more deck sections (not shown), some or all of which be articulating sections, i.e., pivotable with respect to base 8. In general, patient support 10 is to be supported by deck 6.

Patient support 10 has an associated control unit 42, which controls inflation and deflation of certain internal components of patient support 10, among other things, Control unit 42 includes a user interface 44, which enables caregivers, service technicians, and/or service providers to configure patient support 10 according to the needs of a particular patient. For example, support characteristics of patient support 10 may be adjusted according to the size, weight, position, or activity level of the patient. User interface 44 is password-protected or otherwise designed to prevent access by unauthorized persons.

User interface 44 also enables patient support 10 to be adapted to different bed configurations, For example, deck 6 may be a flat deck or a step or recessed deck. A caregiver may select the appropriate deck configuration via user interface 44. Inflation or deflation of specific mattress components may occur in response to user selection of a hospital bed frame or deck configuration.

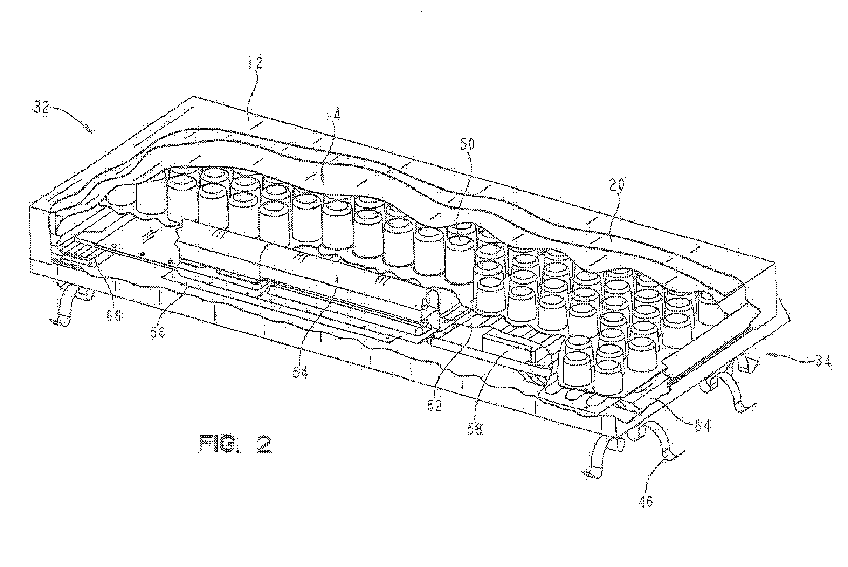

Referring now to Fig. 2, patient support 10 has a head end 32 generally configured to support a patient's head and/or upper body region, and a foot end 34 generally configured to support a patient's feet and/or lower body region. Patient support 10 includes a covet 12 which defines an interior region 14. In the illustrated embodiment, interior region 14 includes a first layer 20, a second layer 50, and a third layer 52. However, it will be understood by those skilled in the art that other embodiments of the present invention may not include all three of these layers, or may include additional layers, without departing from the scope of the present invention.

In the illustrated embodiment, first layer 20 includes a support material, second layer 50 includes a plurality of vertically-oriented inflatable bladders located underneath the first layer 20, and third layer 52 includes a plurality of pressure sensors located underneath the vertical bladders of second layer 50, as more particularly described below.

Also located within interior region 14 are a plurality of bolsters 54, one or more filler portions 56, and a pneumatic valve control box 58. A fire-resistant material (not shown) may also be included in the interior region 14.

Patient support 10 may be coupled to deck 6 by one or more couplers 46. Illustratively, couplers 46 are conventional woven or knit or fabric straps inducing a D-ring or hook and loop assembly or Velcro®-brand strip or similar fastener. It will be understood by those skilled in the art that other suitable couplers, such as buttons, snaps, or tethers may also be used equally as well.

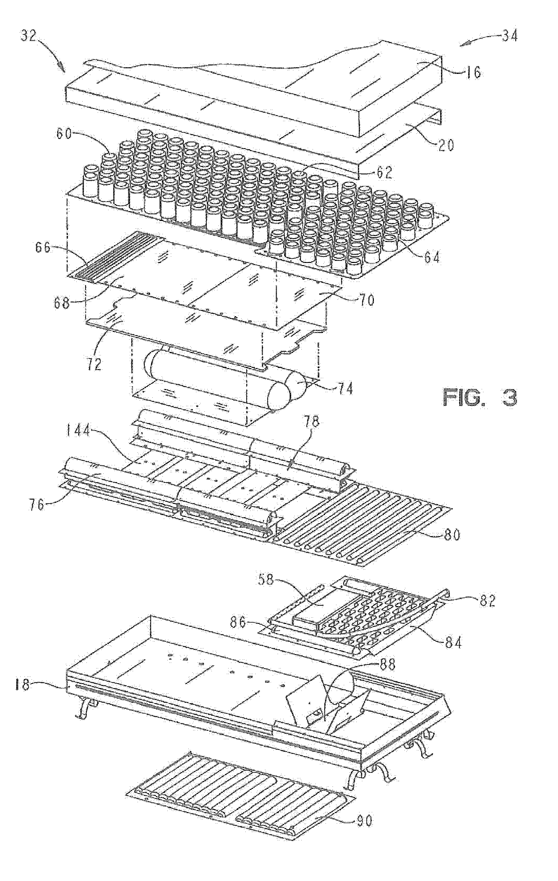

Components of one embodiment of a patient support in accordance with the present invention are shown in exploded view in Fig. 3. This embodiment of patient support 10 includes a top cover potion 16 and a bottom cover portion 18. Top cover portion 16 and bottom cover portion 18 couple together by conventional means (such as zipper, Velcro® strips, snaps, buttons, or other suitable fastener) to form cover 12, which defines interior region 14, While a plurality of layers and/or components are illustrated within interior region 14, it will be understood by those of skill in the art that the present invention does not necessarily require all of the illustrated components so be present.

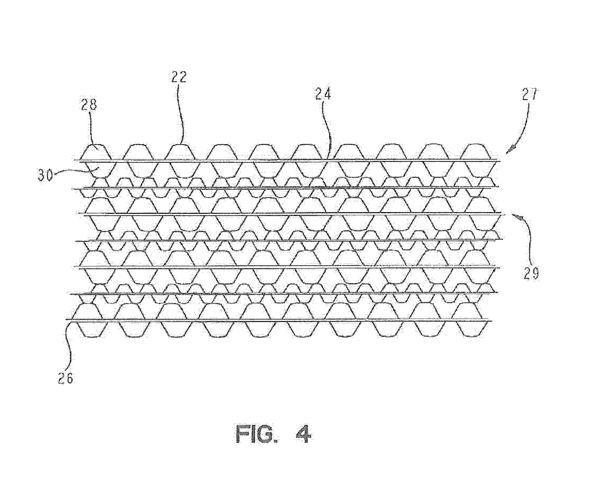

A first support layer 20 is located below top cover potion 16 in interior region 14. First support layer 20 includes one or more materials, structures, or fabrics suitable for supporting a patient, such as foam, inflatable bladders, or three-dimensional material. Suitable three-dimensional materials include Spacenet, Tytex, and/or similar materials. One embodiment of a suitable three dimensional material for support layer 20 is shown in Fig. 4, described below,

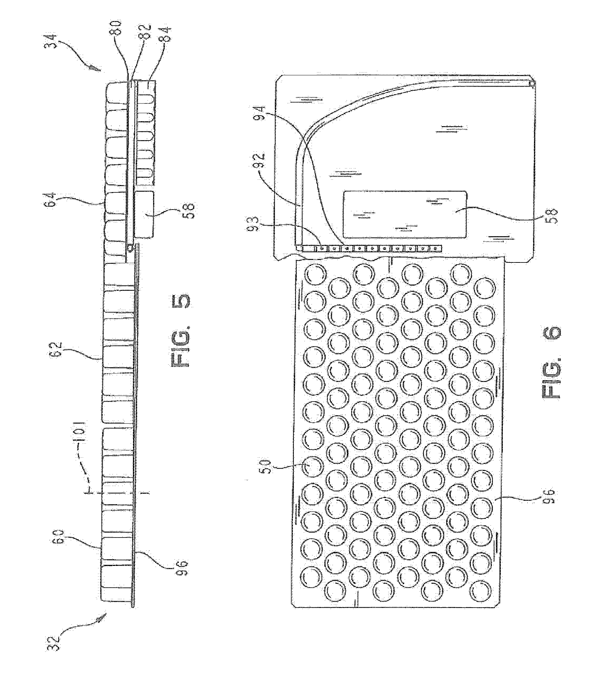

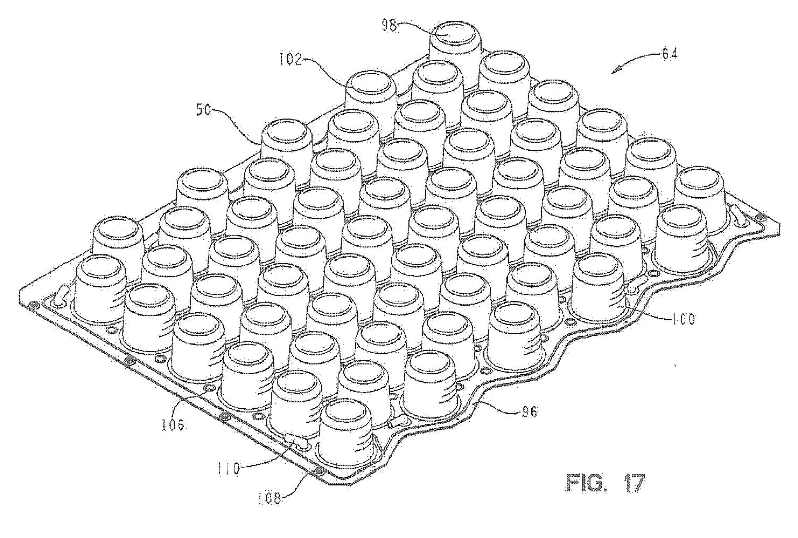

Returning to Fig. 3, a second support layer 50 including one or more inflatable bladder assemblies, is located underneath the first support layer 20. The illustrated embodiment of the second support layer 50 includes first, second and third bladder assemblies, namely, a head section bladder assembly 60, a seat section bladder assembly 62, and a foot section bladder assembly 64. However, it will be understood by those skilled in the art that other embodiments include only one bladder assembly extending from head end 32 to foot end 34, or other arrangements of multiple bladder assemblies, for example, including an additional thigh section bladder assembly. The illustrated bladder assemblies 60, 62, 64 and their components are described below with reference to Figs. 5-19. In general, bladder assemblies disclosed herein are formed from a lightweight, flexible air-impermeable material such as a polymeric material like polyurethane, urethane-coated fabric, vinyl, or rubber.

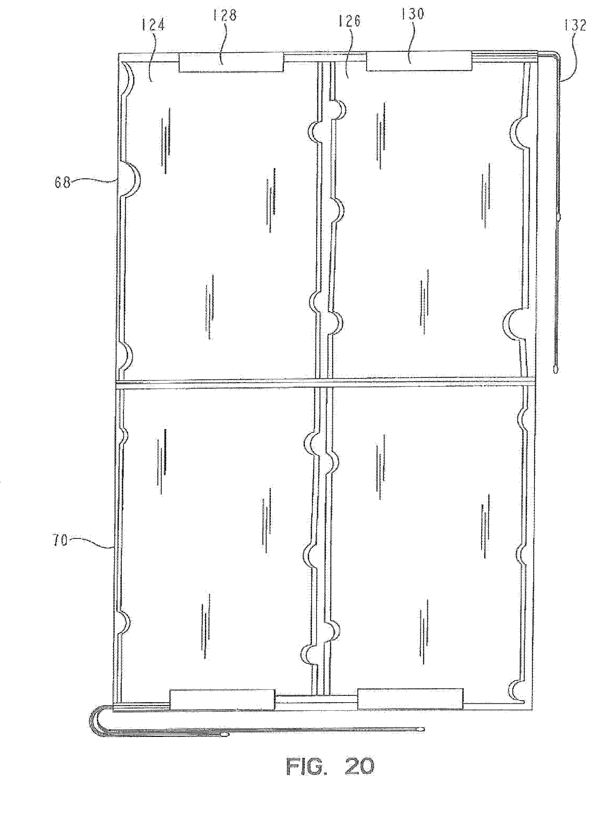

A pressure-sensing layer 69 illustratively including first and second sensor pads, namely a head sensor pad 68 and a seat sensor pad 70, is positioned underneath bladder assemblies 60, 62, 64. Head sensor pad 68 is generally aligned underneath head section bladder assembly 60, and seat sensor pad 70 is generally aligned underneath seat section bladder assembly 62, as shown. Head filler 66 may be positioned adjacent head sensor pad 68 near head end 32 so as to properly position head sensor pad 68 underneath the region of patient support 10 most likely to support the head or upper body section of the patient. In other embodiment, a single sensor pad or additional sensor pads, for example, located underneath foot section bladder assembly 64, and/or different alignments of the sensor pads, are provided. Sensor pads 68, 70 are described below with reference to Figs. 20-21.

In the illustrated embodiment, a turn-assist cushion or turning bladder or rotational bladder 74 is located below sensor pads 68, 70. The exemplary turn-assist cushion 74 shown in Fig. 3 includes a pair of inflatable bladders 74a, 74b. Another suitable rotational bladder 74 is a bellows-shaped bladder. Another suitable turn-assist cushion is disclosed in, for example, U.S. Patent No. 6,499,167 to Ellis, et al., which patent is owned by the assignee of the present invention and incorporated herein by this reference, Turn-assist cushions 74 are not necessarily a required element of the present invention.

A plurality of other support components 66, 72, 76, 78, 80, 84, 86, 90 are also provided in the embodiment of Fig. 3. One or more of these support components are provided to enable patient support 10 to be used in connection with a variety of different bed frames, in particular, a variety of bed frames having different deck configurations. One or more of these support components may be selectively inflated or deflated or added to or removed from patient support 10 in order to conform patient support 10 to a particular deck configuration, such as a step or recessed deck or a flat deck.

The support components illustrated in Fig. 3 are made of foam, inflatable bladders, three-dimensional material, other suitable support material, or a combination of these. For example, as illustrated, head filler 66 includes a plurality of foam ribs extending transversely across patient support 10. Head filler 66 could also be an inflatable bladder, Filler portion 72 includes a foam layer positioned substantially the sensor pads 68, 70 and extending transversely across the patient support 10, In the illustrated embodiment, filler portion 72 includes a very firm foam, such as polyethylene dosed-cell foam, with a ½-inch thickness.

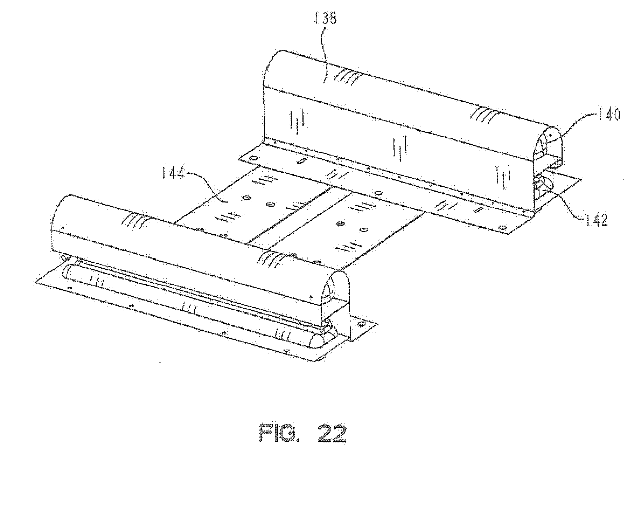

Head bolster assembly 76, seat bolster assembly 78, and foot section bolster assembly 86 each include longitudinally-oriented inflatable bladders spaced by coupler plates 144. Bolster assemblies 76, 78, 86 are described below with reference to Fig. 22.

As illustrated, first foot filler portion 80 a plurality of inflatable bladders extending transversely across patient support 10, and second foot filler portion 84 includes a foam member, with portions cut out to allow for retractability of the foot section or for other reasons, Deck filler portion 90 induces a plurality of transversely-extending inflatable bladders. As illustrated, deck filler portion 90 includes two bladder sections located beneath the head and seat sections of the mattress, respectively, and is located outside of cover 12, Deck filler portion 90 may include one or more bladder regions, or may be located within interior region 14, without departing from the scope of the present invention.

Also provided in the illustrated embodiment are a pneumatic valve box 58 and an air supply tube assembly 82. Receptacle 88 is sized to house pneumatic valve box 58. In the illustrated embodiment, receptacle 88 is coupled to bottom cover potion 18 by Velcro® strips, Pneumatic box 58 and tube assembly 82 are described below with reference to Fig. 6, Fig. 23, and Figs. 25-26.

In the illustrated embodiment, support layer 20 includes a breathable or air permeable material which provides cushioning or support for a patient positioned thereon and allows for circulation of air underneath a patient. The circulated air may be at ambient temperature, or may be cooled or warmed in order to achieve desired therapeutic effects.

Also in the illustrated embodiment, support layer 20 includes or is enclosed in a tow friction air permeable material (such as spandex, nylon, or similar material) enclosure that allows support layer 20 to move with movement of a patient on patient support 10, in order to reduce shear forces, for instance. In other embodiments, the enclosure is made of a non-air permeable, moisture/vapor permeable material such as Teflon or urethane-coated fabric.

In Fig. 4, an exemplary three-dimensional material suitable for use in support layer 20 is depicted. This illustrated embodiment of support layer 20 induces a plurality of alternating first and second layers 27, 29. Each layer 27, 29 includes first and second sublayers 28, 30. As shown, the sublayers 28,30 are positioned back-to-back and each sublayer 28, 30 includes a plurality of peaks or semicircular, cone, or dome-shaped projections 22 and troughs or depressions 24. A separator material 26 is provided between the first and second sublayers 28, 30. In other embodiments, separator material 26 may instead or in addition be provided between the layers 27, 29, or not at all.

Any number of layers and sublayers may be provided as may be desirable in a particular embodiment of support layer 20. Certain embodiments induce 4 layers and other embodiments include 8 layers. In general, 0-20 layers of three dimensional material are included in support layer 20.

Suitable three-dimensional materials for use in support layer 20 include a polyester weave such as Spacenet, manufactured by Freudenberg & Co. of Weinheim, Germany, Tytex, available from Tytex, Inc. of Rhode Island, U.S.A., and other woven, nonwoven, or knit breathable support materials or fabrics having resilient portions, microfilaments, monofilaments, or thermoplastic fibers. Other embodiments of support layers and suitable three dimensional materials are described in U.S. Patent Application Serial No. _____, entitled **PRESSURE RELIEF SUPPORT SURFACE** (Attorney Docket No. 8266-1220), filed on the same date herewith, and assigned to the assignee of the present invention, the disclosure or which is incorporated herein by this reference.

An exemplary second support layer including a base 96 and a plurality of inflatable bladders 50 is shown in the side view of Fig. 5. Inflatable bladders 50 extend upwardly away from base 96 along a vertical axis 101. Inflatable bladders 50 are arranged into a plurality of bladder zones, namely head bladder zone 60, seat bladder zone 62, and foot bladder zone 64. First and second foot filler portions 80, 84 and tube assembly 82 are located in the foot end 34 of patient support 10 below foot bladder assembly 64. Pneumatic valve box 58 is also located in foot end 34 of patient support 10 underneath foot bladder zone 64, In other embodiments, pneumatic box 58 may be located elsewhere in patient support 10 or outside patient support 10.

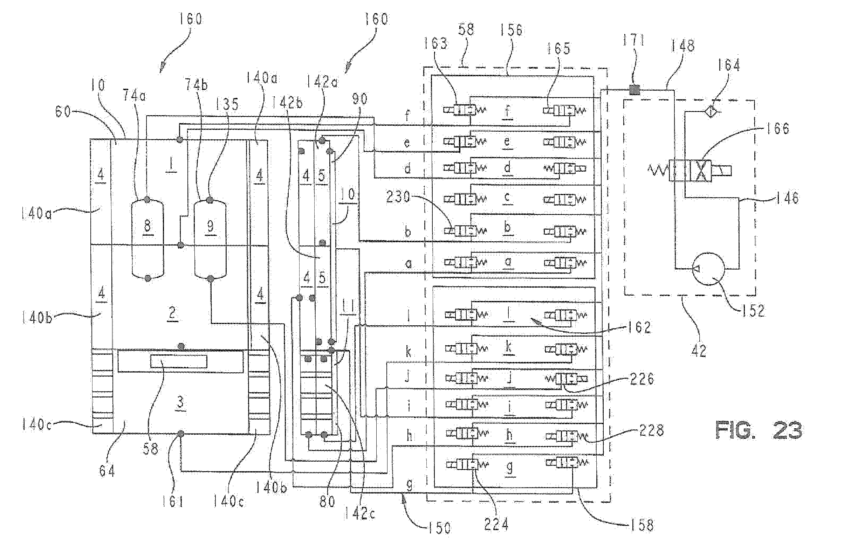

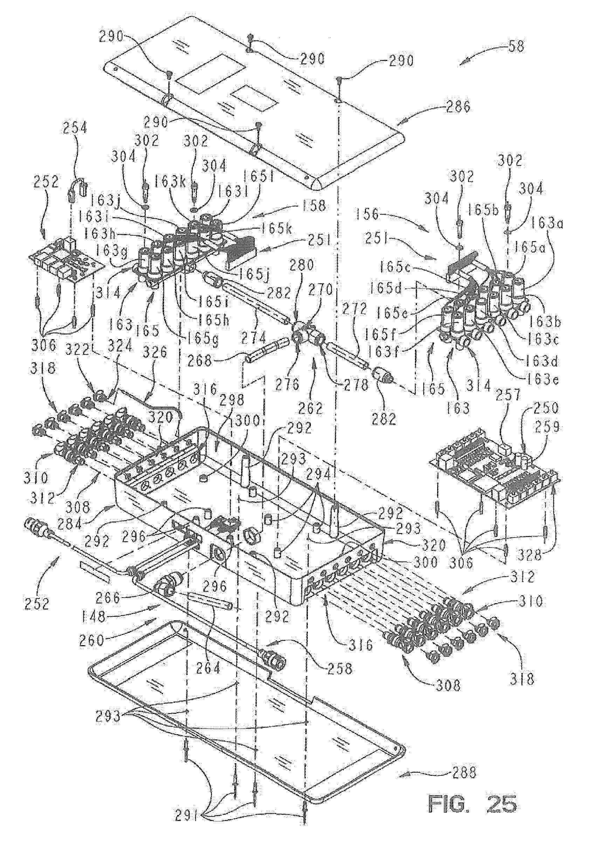

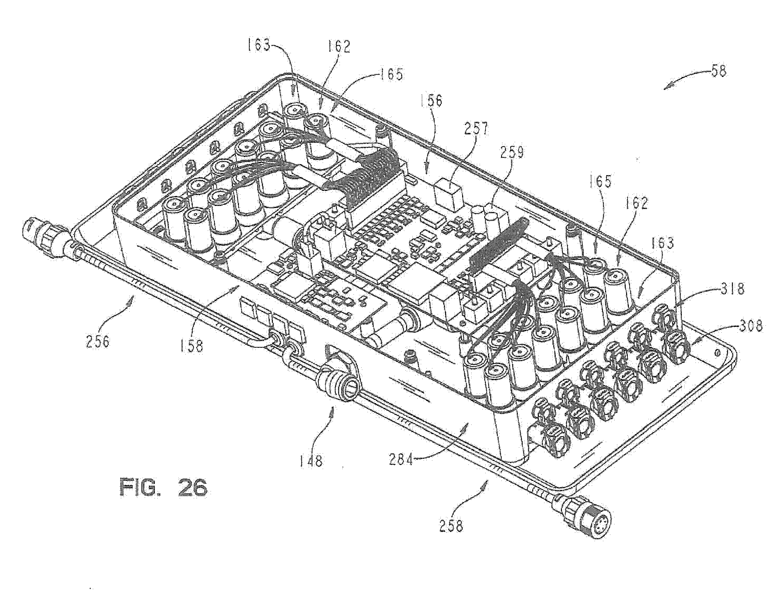

In Fig. 6, a top view of the above-described embodiment of patient support 10 is provided, with cover 12, support layer 20, and foot bladder assembly 64 removed to show the arrangement of delivery tube 92, air distributor 94, and pneumatic box 58 in the foot section 34. Pneumatic box 58 includes valves, circuitry, and other components for connecting vertical bladders 50 to an air supply 152 (Fig. 23) for inflation and deflation of vertical bladders 50. Pneumatic box 58 is described below with reference to Figs. 25 and 26.

Delivery tube 92 is connected to an air supply and provides air to air distributor 94. In the illustrated embodiment, delivery tube extends transversely and/or diagonally across the width of patient support 10 and may be curved or angled toward seat section bladder zone 62. Tube 92 and distributor 94 are made of a lightweight air impermeable material such as plastic.

Air distributor 94 is coupled to an end of delivery tube 92 located near seat section bladder zone 62. Air distributor 94 is an elongated hollow member including one or more apertures 93 which allow air to exit the tube 92 and circulate among vertical bladders 50 and three-dimensional material 20. In certain embodiments, the air is directed upwardly through support layer 20. A vent (not shown) is provided in cover 12 to allow the circulated air to exit interior region 14. The vent is generally located on the opposite end of patient support 10 from the supply tube 92. An additional vent may be provided in the three-dimensional material enclosure, in embodiments where three-dimensional material 20 is enclosed in an enclosure within interior region 14 as discussed above, In those embodiments, the vent is also generally be located opposite the supply tube 92.

In the illustrated embodiment, air provided by delivery tube 92 does not bleed upwardly through cover 12, however, in other embodiments cover 12 may induce a breathable or air permeable material allowing for air to flow upwardly through the cover 12 to the patient. Also, in other embodiments, a single supply tube is provided in place of delivery tube 92 and air distributor 94. While shown in the illustrated embodiment, the above-described air circulating feature is not necessarily a required component of the present invention,

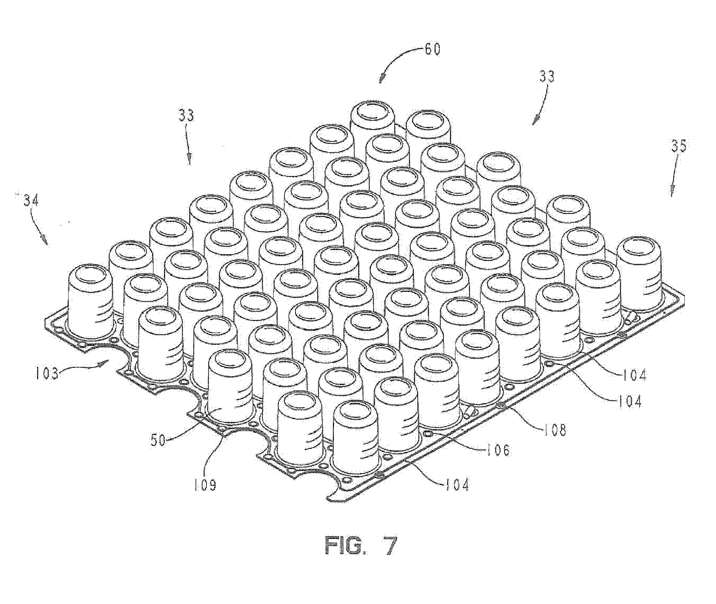

Exemplary vertical bladder assemblies are shown in the perspective views off Figs. 7, 14, 16, and 17. Fig. 7 illustrates a head section bladder assembly 60. Head section bladder assembly 60 includes a base or substrate 96 and a plurality off vertically-oriented inflatable bladders 50 extending upwardly from the base 96 along an axis 101 which is substantially perpendicular to base 96.

Head section bladder assembly 60 has a head end 32, a foot end 34, a first side 33 and a second side 35. Vertical bladders 50 are arranged in longitudinally-extending columns from head end 32 to foot end 34, and in transversely extending rows from first side 33 to second side 35.

Each bladder 50 is coupled to base 96 by a coupling 104. In the illustrated embodiment, radio frequency (RF) welding is used to couple bladders 50 to base 96. In other embodiments, other conventional coupling means, such as adhesives or sealants, may be used.

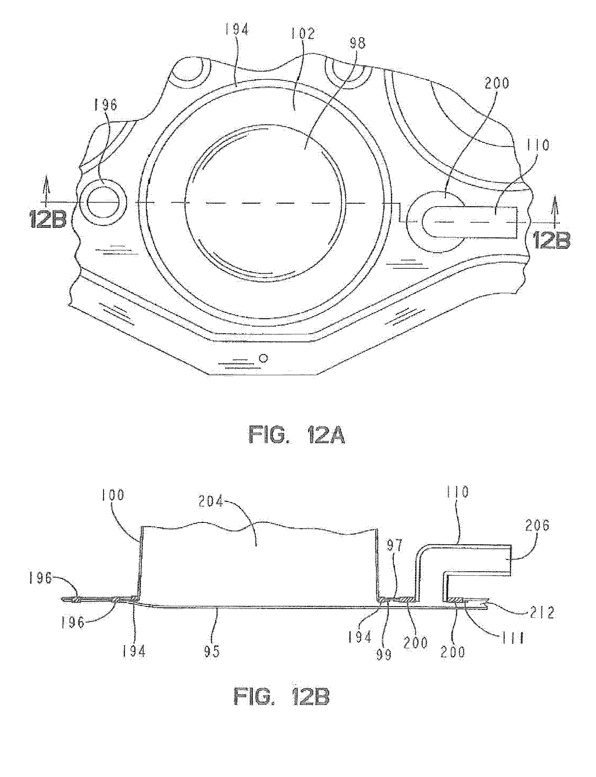

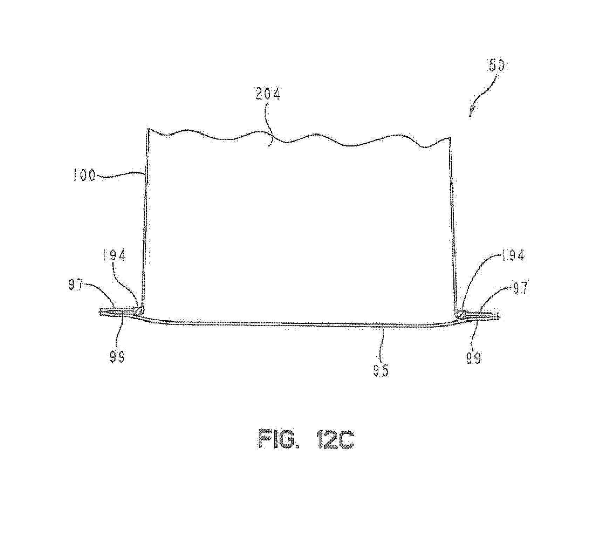

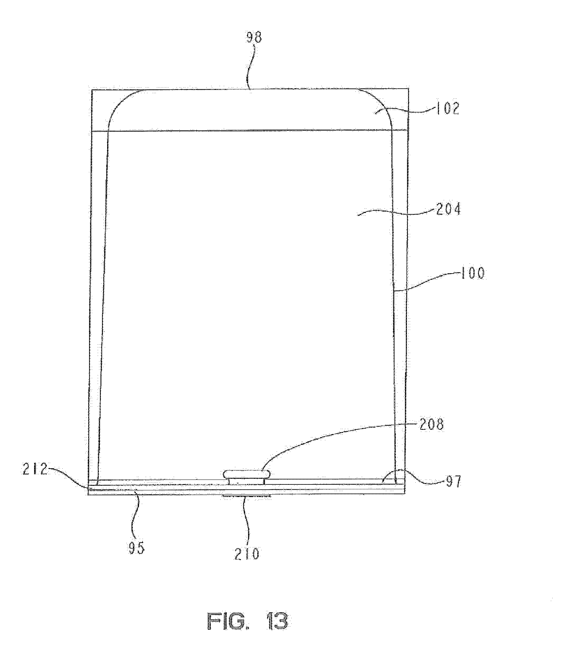

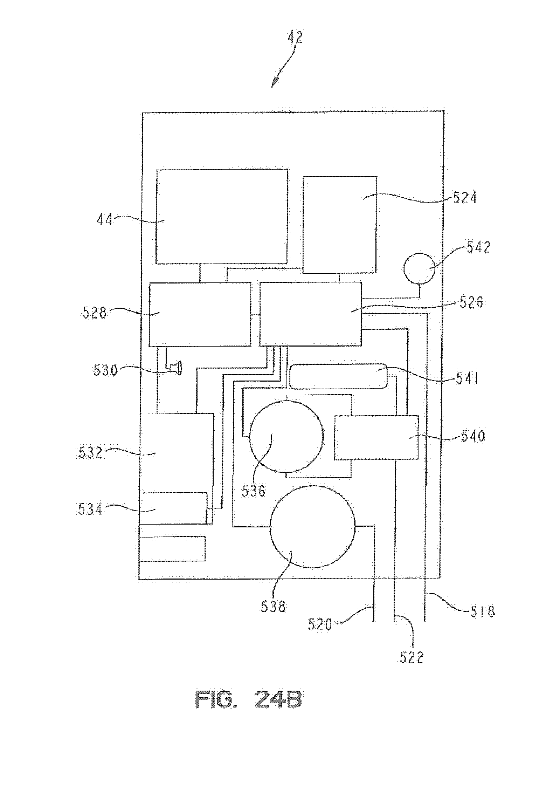

Base 96 includes an upper base portion or substrate 97 and a lower base portion or substrate 95 as best shown in Figs. 12B, 12C and 13. Air channels 212 (best shown in Fig. 12B) are formed between upper base portion 97 and lower base portion 95 and provide air to bladders 50 from an air supply (Fig. 24B). Air release channels are coupled to air channels 212 as shown in Fig. 12B.

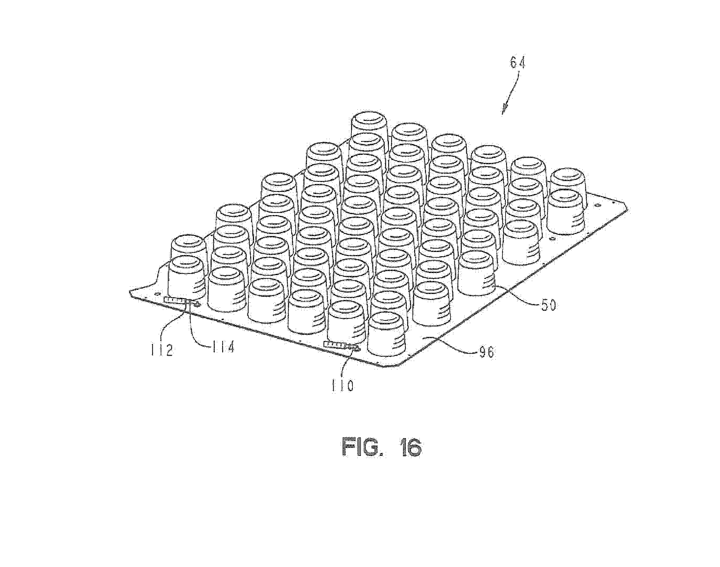

Elbow ports 110 and fittings 114 are coupled to air release channels 206 and to relief valves 112 as shown in Fig. 16. One suitable fitting 114 is a ½ inch by 3/8 inch barbed connector, model no. C8-6WN made of nylon, available from Eldon James Corp. of Loveland, Colorado,

Pressure relief valves 112 release air to the atmosphere, for example, if the internal air pressure within the bladders 50 exceeds a maximum value. One suitable relief valve 112 is a 2.0 psi pressure relief valve model no. 730ROA available from Halkey-Roberts of St. Petersburg, Florida. In the illustrated embodiment, relief values 112 are inserted into tubing such as 1/2 -inch clear PVC tubing.

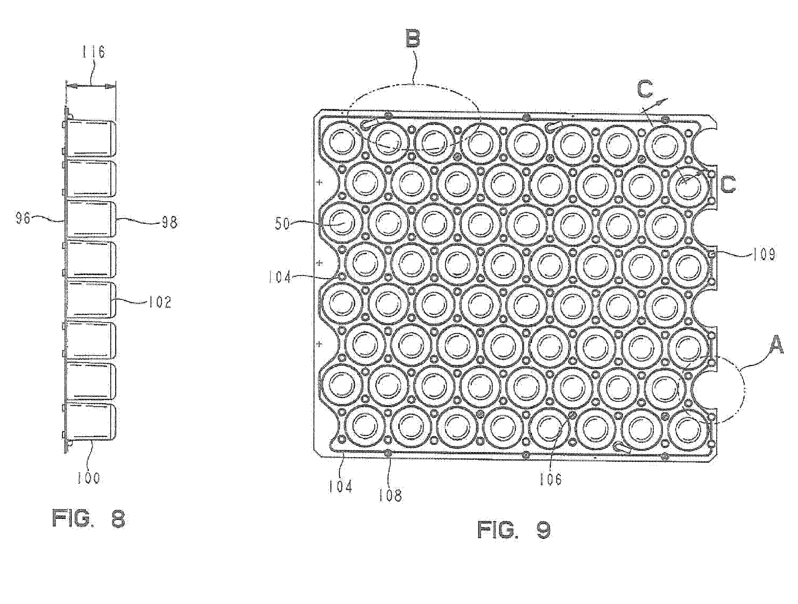

Returning now to Figs. 7 and 9, upper base portion 97 is coupled to lower base portion 95 by welds 104, forming a plenum. Fasteners, 106, 108, 109 are generally spaced apart along the outer edges of base 96, Fasteners 106, 108, 109 secure the bladder assembly 60 within interior region 14, i.e. by coupling bladder assembly 60 to another bladder assembly, or to an inner portion of cover 12, or to stiffener plates 144, or to component within interior region 14, As shown, fasteners 106 couple the bladder assembly 60 to the sensor pad 68, fasteners 108 couple bladder assembly 60 to support plate 144, and fastener 109 couple bladder assembly 60 to bladder assembly 62. Fasteners 106, 108, 109 are, in the illustrated embodiment, plastic or metal buttons and snaps. However, other suitable fasteners, such as rivets, may also be used.

Elbow ports 110 are spaced apart and located along the edges of first and second sides 33, 35 of bladder assembly 60. In the illustrated embodiment, 3/8-inch pods 110 are provided on each side of bladder assembly 60 and at least one port is provided for each of head section assembly 60, seat section assembly 62, and foot section assembly 64.

Semi-circular regions 103 facilitate coupling of head section bladder assembly 60 with another bladder assembly, among other things, For instance, semi-circular regions 103 are sized to mate with vertical bladder portions of another bladder assembly, such as seat section bladder assembly 62.

Fig. 8 is a side view of bladders 50 of head section bladder assembly 60. bladders 50 each have a vertical portion 100, a top portion 98, and an angled oar beveled portion 102 located in between the top portion 98 and the vertical portion 100. Each bladder 50 is coupled to base 96. Bladders 50 have a vertical height 116. in certain embodiments, vertical height 116 of bladders in the head section bladder assembly 60 is about 4 to about 6 inches. In one embodiment, vertical height 1 16 is about 4.9 and the height including plenum 95, 97 is about 6 inches.

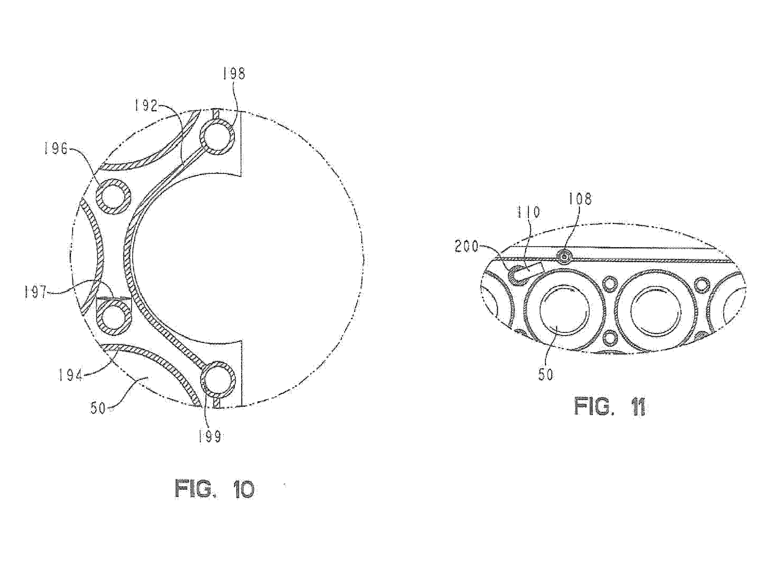

Fig. 9 shows the pattern of couplings 104 (i.e., RF welds) used in the head section bladder assembly 60 of the illustrated embodiment of the present invention, Radio frequency welds 104 are provided around the circumference of each bladder 50, intermittently between the bladders 50 on the base 96, and along the outer edges of the bladder assembly, Welds 194 around the circumference of each bladder 50 couple coupling portion 99 of bladder 50 to upper base portion 97 as best shown it Figs. 10, 11, 12B and 12C. Welds 196, 198 couple upper base portion 97 to lower base portion 95 as best shown in Figs. 10, 11, and 12B. Dielectric welds 105 are used to join elbow ports 110 to upper base portion 95 as best shown in Figs, 11 and 12B.

Fig. 10 is a close-up view of Area A of Fig. 9, showing outer edge welds 192, bladder welds 194, first circular welds 196, and second circular welds 198. In the illustrated embodiment, the welds 198 have an outer diameter 197 of about 4 inches and a thickness of about . 125 inches; welds 196, 198 have an outer diameter off about .75 inches and a thickness of about. 125 inches; and welds 192 have a thickness of about. 125 inches. Other suitable weld configurations may be used without departing from the scope of the present invention, In some cases, circular welds 196, 198 may encircle fasteners 106, 108.

Fig. 11 additionally shows a weld 200 encircling an elbow port 110, which, us mentioned above, is done by dielectric welding.

Fig. 12A illustrates a of bladder assembly 60 including a weld 196, a bladder 50, and an elbow port 110. As discussed above, bladder 50 includes a top portion 98 and a beveled portion 102. Bladder 50 is substantially cone-or can-shaped or cylindrical in shape. However, in other embodiments, bladder 50 is square or cube-shaped, rectangular, hexagonal, octagonal, or any other suitable shape as will be understood by those of ordinary skill in the art. Welds 196, 194, and 200 are generally circular in shape. In the case of bladder weld 194, weld 194 is positioned along the circumference of bladder 50. In the case of weld 200, weld 200 is positioned along the circumference of elbow port 110.

Fig. 12B shows a cross sectional view of Fig. 12A taken along line 12B-12B. As shown in Fig. 12B, an air channel 212 supplies air from an air supply (not shown) to interior region 204 of bladder 50. Some of the air provided by air channel 212 resides in release channel 206, If the pressure in interior region 204, 206, 212 exceeds a maximum value, a pressure relief value 112 coupled to channel 206 will release air to the atmosphere.

Air channel 212 is formed between upper base portion 97 and lower base portion 95, which are coupled together at welds 196. Upper base portion 97 includes a plurality of cut-out regions or holes (not shown) into which material forming bladders 50 is inserted, Each bladder 50 has an end portion 99 that is positioned between upper base portion 97 and lower base portion 95 as shown in Figs. 12C and 13. End portion 99 is secured to upper base portion 97 by coupling or weld 194. Similarly, release channel 206 includes an end portion 111 which is secured to upper base portion 97 by coupling or weld 200.

In the illustrated embodiment, bladder 50 is thermoformed and only welded where end portion 99 meets upper base portion 97. In other embodiments, bladder 50 may be hand-crafted; i.e., top portion 98 is welded to vertical portion 100 and vertical portion 100 also includes a welded seam.

Another cross-section of a portion of a bladder assembly 60 is shown in Fig. 13. Fig. 13 depicts an exemplary fastener 106, 108 suitable for coupling upper base portion 97 to lower base portion 95, and or for coupling base 96 to another surface (such as bottom surface of cover 12) within interior region 14, In the illustrated embodiment, each fastener 106, 108 includes a first fastener portion or stud 208 and a second fastener portion or post 210. In other embodiments, first fastener portion 208 is a button and second fastener portion 210 is a socket. In any case, first and second fastener portions 208, 210 are configured to mate with one another thereby coupling the intervening materials together.

Fig. 13 also illustrates air channel 212 located between upper and lower base portions 97, 95, vertical bladder wall 100 extending upwardly away from base 96, bladder top portion 98, and crown or bevel portion 102 located between top portion 98 and wall portion 100, defining an interior bladder region 204. In the illustrated embodiment, the diameter of each vertical bladder 100 is about 3.5". A smaller or larger diameter may be used in other embodiments.

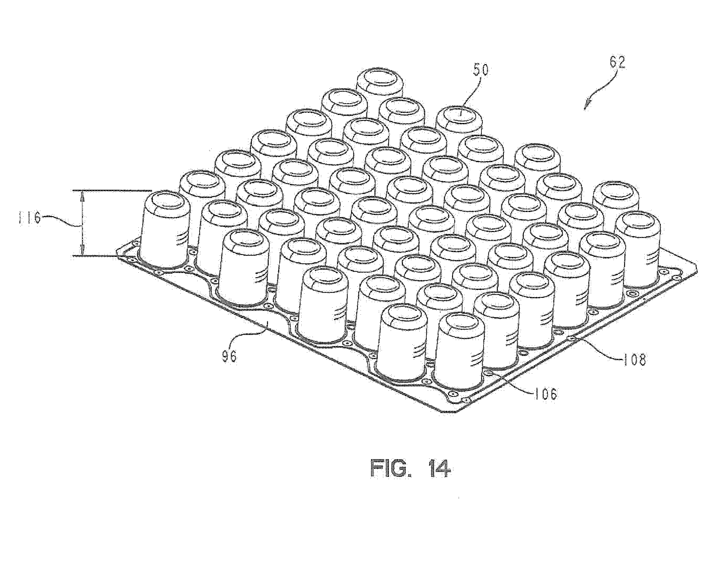

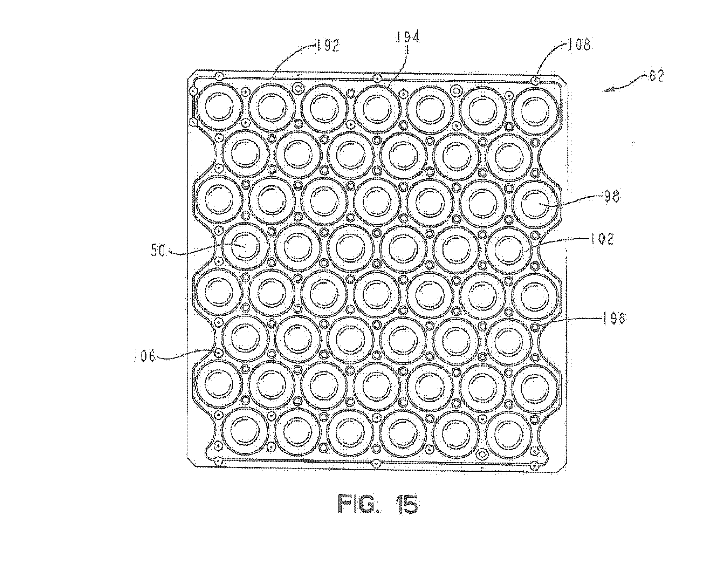

Figs. 14 and 15 depict an exemplary seat section bladder assembly 62. Seat section bladder assembly is generally configured to support a patient's seat, thighs, or midsection. The components of seat section bladder assembly 62 are substantially as described above with reference to head section bladder assembly 60. Bladders 50 of seat section bladder assembly 62 have a vertical height 116. In the illustrated embodiment, vertical height 116 is the same or about the same as vertical height 116 of the bladders in head section assembly 60.

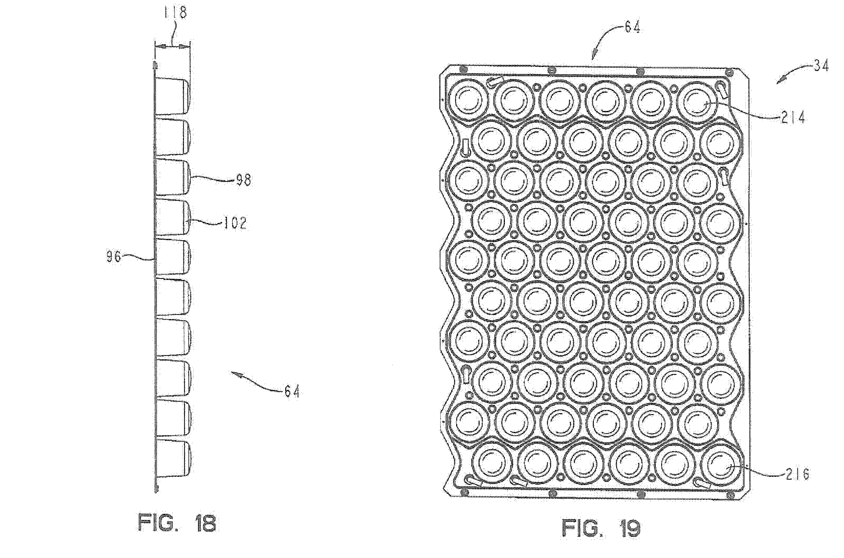

Figs. 16-19 depict an exemplary foot section bladder assembly 64. Foot section bladder assembly 64 is generally configured to support a patient's legs and/or feet. The components of foot section bladder assembly 64 are substantially as described above with reference to head section bladder assembly 60. bladders 50 of foot section bladder assembly have a vertical height 118 as shown in Fig. 18. In the illustrated embodiment, vertical height 118 is shorter or smaller than vertical height 116. In certain embodiments, vertical height 118 is about 2 to about 5 inches. In one embodiment, vertical height is about 3.5 inches and the height including plenum 95, 97 is about 4 inches.

As shown in Fig. 19, the illustrated embodiment of foot section bladder assembly 64 is wider than head and seat section bladder assemblies 60, 62. In this embodiment, foot section bladder assembly 64 includes longitudinal sections 214, 216, which may function as bolsters for the foot section, Longitudinal sections 214, 216 are separately inflatable from the remainder of the foot section 64, in the illustrated embodiment.

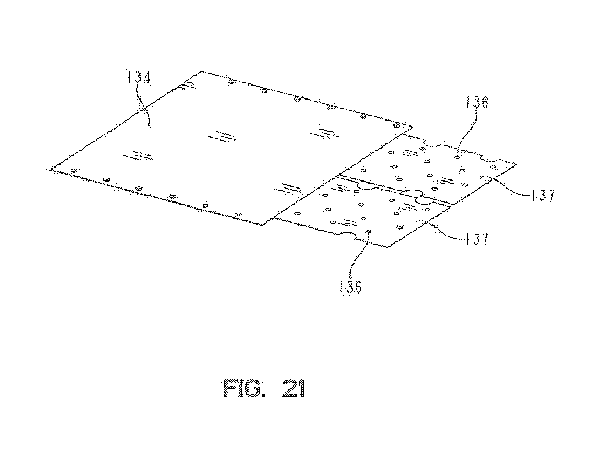

In the illustrated embodiment, patient support 10 includes a pressure sensing member 67 located underneath the bladder assemblies 60, 62, 64. As shown in Fig. 20, pressure sensing member 67 includes a first or head section sensor pad 68 and a second or seat section sensor pad 70. Further, each sensor pad 68, 70 includes first and second sensor pad portions 124, 126. Each sensor pad portion 124, 126 includes a plurality of pressure sensors 136 located on a substrate 220. Sensors 136 are designed to respond when pressure is applied to the top surface of patient support 10, i.e. by a patient. Sensors 136 are spaced apart and arranged on substrate 220 so that they are positioned adjacent or underneath or aligned with one or more vertical bladders 50. In the illustrated embodiment, a sensor 136 is positioned underneath the center or middle portion of each vertical bladder 50. Substrate 220 is made of a substantially rigid material such as plastic. An additional interface layer may be provided between base 96 and substrate 220 to direct applied force to sensors 136, or for othe reasons. Sensors 136 include a soft or flexible material such as foam and one or more light conductors or optical fibers (not shown) located within the foam. Sensors 136 and substrate 137 are enclosed within a cover 22 as shown in Fig. 21.

The sensors 136 in each sensor pad 68,70 are coupled to a collector/transmitter 128, 130, which receives pressure data from sensors 136 and transmits the data to a circuit located in pneumatic box 58 by communication lines 132. A collector/transmitter 128, 130 is located at one end of each sensor pad portion 124, 126.

In the illustrated embodiment, the pressure data obtained by sensors 136 is indicative of an amount of pressure being applied (i.e., by a patient or portion thereof being supported by patient support 10) to one or more vertical bladders 50. The pressure sensing apparatus 67 and components and operation thereof are described in more detail in U.S. Patent Application Serial Number _____, titled **PATIENT SUPPORT HAVING REAL TIME PRESSURE CONTROL** (Attorney Docket No. 8266-1287), filed on the same date herewith and assigned to the assignee of the present invention, which is incorporated herein by this reference.

Fig. 22 depicts a bolster assembly 76, 78, Bolster assemblies 76, 78 are generally configured to support portions of a patient along the longitudinal edges of patient support 10, One or more bolster assemblies 76, 78 may be provided in order to conform patient support 10 to a particular bed frame configuration, to provide additional support along the edges of patient support 10, aid in ingress or egress of a patient from patient support 10, maintain a patient in the center region of patient support 10, or for other reasons. For example, internal air pressure of the bolster bladders may be higher than the internal bladder pressure of assembles 60, 62, 64, or may be increased or decreased in real time, to accomplish one of these or other objectives.

Each bolster assembly 76,78 includes a plurality of bolsters, namely, an upper bolster 140 and a lower bolster 142, with the upper bolster 140 being positioned above the lower bolster 142. Each upper and lower bolster combination 140, 142 is configured to be positioned along a longitudinal edge of patient support 10. Each upper and lower bolster combination 140, 142 is enclosed in a cover 138.

In the illustrated embodiment, the bolsters 140, 142 are inflatable bladders. In other embodiments, either or both bolsters 140, 142 may be constructed of foam, or filled with three-dimensional material, fluid, or other suitable support material. For example, in one embodiment, upper bolster 140 includes two layers of foam: a viscoelastic top layer and a non visco elastic bottom layer, while lower bolster 142 is an inflatable bladder. The bolsters 140, 142 may be inflated together, or separately, as shown in Fig. 23, described below.

Each bolster combination 140, 142 is coupled to one end of one or more support plates 144 which provide support for other components of patient support 10 including vertical bladders 50. Support plates 144 may be made of a substantially rigid or stiff yet lightweight material such as molded plastic. In other embodiments, plates 144 may be constructed of stainless steel or steel, if additional weight is desired, i.e. for addition, collapsibility for ease of storage of patient support 10, for instance. Support plates 144 may be provided in order to give support to patient support 10 particularly during transport, for ease of assembly, or for other reasons.

In the illustrated embodiment, each support plate 144 is a rectangular member extending transversely across the width of the mattress 10. As shown in the drawings, there are five such rib-like members 144 spaced apart underneath the head and seat sections of the mattress. In other embodiments, each support plate 144 has its middle section (i.e., the section extending transversely) cut out so that only the two plate ends remain at each spaced-apart end (underneath the bolsters); thereby providing five pairs of support plates 144 spaced apart along the longitudinal length of the mattress 10.

Bolster assembly 86 is similar to bolster assemblies 76, 78 except that its upper layer includes the vertical bladders 50 of longitudinal sections 214, 216. Bolster assembly 86 has a longitudinally-oriented bladder as its lower bolster portion.

A schematic diagram of the pneumatic control system of patient support 10 is shown in Fig. 23. Reading Fig. 23 from second to first, there is shown a simplified top view of patient support 10 with portions removed to better illustrate the various air zones 160, a simplified side view of patient support 10, a schematic representation of pneumatic valve box 58, a schematic representation of control unit 42, and air lines 146, 148, 150 linking control unit 42, valve box 58, and air zones 160.

As shown in Fig. 23, air zones 160 of patient support 10 are assigned as follows: zone 1 corresponds to head section bladder assembly 60, zone 2 corresponds to seat section bladder assembly 62, zone 3 corresponds to foot section bladder assembly 64, zone 4 corresponds to upper side bolsters 140, zone 5 corresponds to lower side bolsters 142, zone 6 corresponds to upper foot bolsters 140, tone 7 corresponds to lower foot bolsters 142, zone 8 corresponds to first turn-assist bladder 74, zone 9 corresponds to second turn-assist bladder 74, zone 10 corresponds to deck filler 90, and zone 11 corresponds to foot filler 80.

An air line 150 couples each lone 160 to a valve assembly 162 in valve box 58. Valve box 58 is located in the foot section 34 of patient support 10. Illustratively, valve box 58 is releasably coupled to bottom portion 18 of cover 12 in interior region 14, i.e., by one or more Vecro®-brand fasteners or other suitable coupler.

Each air line 150 is coupled at one end to an inlet port 135 on the corresponding bladder or bladder assembly. Each air line 150 is coupled at its other end to a valve assembly 162. Each valve assembly 162 includes first or fill valve 163 and a second or vent valve 165. First valves 163 are coupled to air supply 152 of control unit 42 by air liners 148. First valves 163 thereby operate to control inflation of the corresponding zone 160 i.e. to fill the zone with air. Second valves 165 operate to at least partially deflate or vent the corresponding zone 160, for example, if the internal air pressure of the zone 160 exceeds a predetermined maximum, or if deflation is necessary or desirable in other circumstances (such as a medical emergency, or for transport of patient support 10).

Each valve 163, 165 has an open mode 224 and a closed mode 226, and a switching mechanism 228 (such as a spring) that switches the value from one mode to another based on control signals from control unit 42. In closed mode 226, air flows from air supply 152 through the value 163 to the respective zone 160 to inflate the corresponding bladders, or in the case of vent valves 165, from the zone 160 to atmosphere. In open mode 228, no inflation or deflation occurs.

In the illustrated embodiment, an emergency vent valve 230 is provided to enable quick deflation of turning bladders 74 which draws air from atmosphere through a filter 164 and also vents air to atmosphere through filler 164. Air supply 152 is an air pump, compressor, blower, or other suitable air source.

Air supply 152 is coupled to a switch valve 155 by air line 146. Switch valve 166 operates to control whether inflation or deflation of a zone occurs. An optional proportional valve 171 may be coupled to air line 148 to facilitate smooth inflation or deflation of turn-assist bladders 74, or for other reasons.

In the illustrated embodiment, valve box 58 includes a first valve module 156 and a second valve module 158. First valve module 156 includes valves generally associated with a patient's first side (i.e., first side, from the perspective of a patient positioned on patient support 10) and second valve module 158 included valves generally associated with a patient's second side (i.e., second side).

The various zones 160 are separately inflatable. Certain of the zones 160 are inflated or deflated to allow patient support 10 to conform to different bed frame configurations. For example, the deck filler 90 (zone 10 in Fig. 23) is inflated to conform patient support 10 to certain bed frame configurations, such as step deck configurations including the TotalCare® and CareAssist® bed frames, made by Hill-Rom, Inc., the assignee of the present invention, but is deflated when patient support 10 is used with a flat deck bed frame, such as the Advanta® bed made by Hill-Rom, Inc. As another example, the foot filler 80 (zone 11 in Fig. 23) is inflated when patient support 10 is used with the VersaCare®, TotalCare®, or CareAssist® beds, but the lower side bolsters 142 (zone 5 in Fig. 23) are not inflated when patient support 10 is used with a VersaCare® bed. As still another example, the lower foot bolsters 142 (zone 7 in Fig. 23) are inflated when patient support 10 is used on flat decks or other bed frames, including the Advanta® and VersaCare® bed frames made by Hill-Rom, Inc.

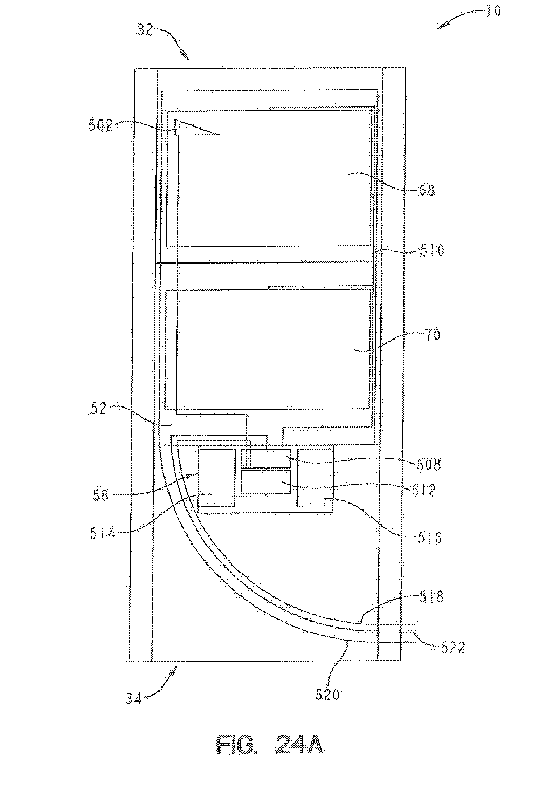

Figs. 24A and 24B are a simplified schematic diagram of a control system and the patient support or mattress 10 of the present invention. Fig. 24A illustrates the patient support 10 including the various components of patient support 10 whereas Fig. 24B illustrates the control unit 42 and various components therein. The patient support 10 includes the sensor pad 52 which is coupled to the pneumatic valve control box 58 as previously described. The sensor pad 52 includes a head sensor pad 68 and a seat sensor pad 70. The head sensor pad 68 is located at the head end 32 of the mattress 10. The seat sensor pad 70 is located at a middie portion of the mattress 10 which is located between the head end 32 and a location of the pneumatic valve control box 58. The seat sensor pad 70 is located such that a patient laying upon the mattress 10 may have its middle portion or seat portion located thereon when in a reclined state. In addition, when the head end 32 of the mattress 10 is elevated, the seat portion of the patient is located upon the seat sensor pad 70. As previously described with respect to Fig. 3, the head sensor pad 68 is located beneath the head section bladder assembly 60 and the seat sensor pad 70 is located beneath the seat section bladder assembly 62. Each one of the sensors of the head sensor pad 68 or the seat sensor pad 70 is located beneath on at least adjacent to one of the upstanding cylindrical bladders or cushions 50. A head angle sensor 502 is coupled to the control box 58 where signals received from the sensor 52 may provide head angle information and pressure adjustment information for adjusting pressure in the seat bladders 62.

The sensor pad 52 is coupled through the associated cabling to the pneumatic control box 58. The pneumatic control box 58 includes a multiplexer 508 coupled to the head sensor pad 68 and the seat sensor pad 70 through a signal and control line 510. The multiplexer board 508 is also coupled to an air control board 512 which is in turn coupled to a first valve block 514 and a second valve 516. A communication/power line 518 is coupled to the control unit 42 of Fig. 24B. Likewise, a ventilation supply line 520 which provides for air flow through the patient support 10 for cooling as well as removing moisture from the patient is also coupled to the control unit 42 of Fig. 24B. An air pressure/vacuum supply line 522 is coupled to the control unit 42 as well.

The control unit 42 of Fig. 24B, also illustrated in Fig. 1, includes the display 44, which displays user interface screens, and a user interface input device 524 for inputting to the control unit 42 user selectable information, such as the selection of various functions or features of the present device. The selections made on the user interface input device 524 control the operation of the patient support 10, which can include selectable pressure control of various bladders within the mattress 10, control of the deck 6, for instance to put the bed 2 in a head elevated position, as well as displaying the current state of the mattress or deck position, and other features.

An algorithm control board 526 is coupled to the user interface input device 524. The algorithm control board 526 receives user generated input signals received through the input device 524 upon the selection of such functions by the user. The input device 524 can include a variety of input devices, such as pressure activated push buttons, a touch screen, as well as voice activated or other device selectable inputs. The algorithm control board 526 upon receipt of the various control signals through the user input device 524 controls not only the operation of the mattress 10 but also a variety of other devices which are incorporated into the control unit 42. For instance, the algorithm control board 526 is coupled to a display board 528 which sends signals to the display 44 to which it is coupled. The display board 528 is also connected to a speaker 530 which generates audible signals which might indicate the selection of various features at the input device 24 or indicate a status of a patient positioned on patient support (e.g. exiting) or indicate a status of therapy being provided to the patient (e.g., rotational therapy complete). The algorithm control board 526 receives the required power from power supply 532 which includes an AC input module 534, typically coupled to a wall outlet within a hospital room.

The algorithm control board 526 is coupled to an air supply, which, in the illustrated embodiment includes a compressor 536 and a blower 538. Both the compressor 536 and the blower 538 receive control signals generated by the algorithm control board 526. The compressor 536 is used to inflate the air bladders. The blower 538 is used for air circulation which is provided through the ventilation supply line 520 to the mattress 10. It is, however, possible that the compressor 536 may be used to both inflate the bladders and to circulate the air within the mattress 10. A pressure/vacuum switch valve 540 is coupled to the compressor 536 which is switched to provide for the application of air pressure or a vacuum to the mattress 10. A muffler 541 is coupled to the valve 540. In the pressure position, air pressure is applied to the mattress 10 to inflate the mattress for support of the patient. In the vacuum position, the valve 540 is used to apply a vacuum to the bladders therein such that the mattress may be placed in a collapsed state for moving to another location or for providing a CPR function, for example. A CPR button 542 is coupled to the algorithm control board 526.

As illustrated, the algorithm control board 526, the compressor 536, the blower 538, and the user input device or user control module 524 are located externally to the mattress and are a part of the control unit 42, which may be located on the footboard 38 as shown in Fig. 1. The sensors and sensor pad 52, the pneumatic valve control box 58, and the air control board or microprocessor 512 for controlling the valves and the sensor pad system 52 are located within the mattress 10. It is within the present scope of the invention to locate some of these devices within different sections of the overall system, for instance, such that the algorithm control board 526 could be located within the mattress 10 or the air control board 512 could be located within the control unit 42.

As shown in Figs. 25-26, control box 58 includes a multiplexer 252 and an air control board 250. Control board 250 is coupled to multiplexer 252 by a jumper 254. Multiplexer 252 is further coupled to head sensor pad 68 and seat sensor pad 70 through a signal and control line (not shown). Control board 250 is also coupled to first valve module 156 and second valve module 158 by wire leads 251. A communication/power line 258 couples control board 250 to the control unit 42. Communication line 258 couples to a communication plug 259 of control board 250. Jumper 254 couples multiplexer 252 to control board 250 for power and access to communication line 258. Wire leads 251 provide actuation power to first and second valve modules 156, 158.

An angle sensor cable 256 is provided to send a signal from a head angle sensor 502 to the control board 250. Angle sensor cable 256 couples to an angle plug 257 of control board 250. In the illustrated embodiment, head angle sensor 502 is located within head bolster assembly 76 as indicated by Fig. 24A. Head angle sensor 502 indicates the angle of elevation of the head end 32 of bed 2 as the head section of the frame 4 articulates upwardly raising the patient's head or downwardly lowering the patient's head. In one embodiment, angle sensor 502 transmits the angle of head end 32 to all nodes or circuit boards within the mattress control system 42, 58. Angle sensor 502 generates an indication or indicator signal when head end 32 is at an angle of at least 5°, at least 30°, and at least 45°. The head angle indication is transmitted to the control unit 42 which evaluates and processes the signal. When head end 32 is at an angle above 30° turn assist 74 becomes inoperative primarily for patient safety reasons. When head end 32 is at an angle above 45° information is transmitted to control unit 42 for use in the algorithms. The 5° angle indication is primarily to ensure relative flatness of patient support 10. In the illustrated embodiment, angle sensor 502 is a ball switch or string potentiometer.

As discussed above, first and second valve modules 156, 158 include fill valves 163 and vent valves 165. First valve module 156 includes fill valves 163a-f and vent valves 165a-f. Second valve module 156 includes fill valves 163g-l and vent valves 165g-l. Fill valves 163a-l and vent valves 165a-l are 12 Volt 7 Watt solenoid direct active poppet style valves in the illustrated embodiment. Control board 252 is able to actuate each fill valve 163a-l and vent valve 165a-l independently or simultaneously. Fill valves 163a-l and vent valves 165a-l are all able to be operated at the same time. In operation to initiate each valve 163, 165, control board 250 sends a signal to the valve to be operated. The signal causes a coil (not shown) within each valve to energize for ½ second and then switches to pulsate power (i.e., turn on and off at a high rate) to save power during activation. The activation in turn cause the valve to either open or close depending on which valve is initiated.

Fill valves 163 are coupled to air supply 152 of control unit 42 by second air line 148. Air line 148 includes an outer box line assembly 260 and an inner box line assembly 262. Outer box line assembly 260 includes an exterior inlet hose 264 and an elbow 266 coupled to exterior inlet hose 264. Inner box line assembly 262 includes an interior inlet hose 268 coupled to elbow 266, a union tee connector 270, a first module hose 272, and a second module hose 274. Connector 270 induces a first opening 276 to receive interior inlet hose 268, a second opening 278 to receive first module hose 272, and a third opening 280 to receive second module hose 274. First and second module hoses 272, 274 each couple through a male coupler 282 to first and second valve modules 156, 158 respectively. In operation, air from air supply 152 travels through supply line 148, enters outer box line assembly 260 through exterior inlet hose 264 and passes through elbow 266 to interior inlet hose 268. The air then travels from inlet hose 268 to union tee connector 270 where the air is divided into first module hose 272 and second module hose 274. The air passes through first and second module hoses 272. 274 into first and second valve modules 156, 158 respectively. The operation of first and second valve modules 156,158 is described below.

Control box 58 includes a base 284, a cover 286, and a tray 288. Cover 286 includes a plurality of fasteners (i.e., screws) 290. Base 284 includes a plurality of threaded cover posts 292. Cover posts 292 are configured to receive screws 290 to couple cover 286 to base 284. Cover 286 and base 284 define an inner region 298. Tray 288 couples to base 284 with a plurality of rivets 291 riveted through a plurality of rivet holes 293 located on tray 288 and base 284.

Inner box line assembly 262, first valve module 156, second valve module 158, control board 250, and multiplexer 252 are contained within inner region 298. Base 284 further includes a plurality of control board posts 294, a plurality of multiplexer posts 296, and a plurality of module posts 300. First and second valve modules 156, 158 are coupled to module posts 300 by shoulder screws 302 and washers 304. Control board 250 and multiplexer 252 are respectively coupled to control board posts 294 and multiplexer posts 296 by a plurality of snap mounts 306.

First and second valve modules 156, 158 attach to third air lines 150 a, b, d-f, and g-l through a plurality of couplers 308. Couplers 308 include a first end 310 and a second end 312. Third air lines 150 a, b, d-f, and g-l each include a fitting (not shown) receivable by second end 312. Each first end 310 mounts to a port 314 in first and second valve modules 156, 158. First end 310 mounts through a plurality of openings 316 in base 284,

A plurality of feedback couplers 318 mount through a plurality of feedback openings 320 in base 284. Feedback couplers 318 include a first feedback end 322 and a second feedback end 324. First feedback end 322 couples to a feedback line (not shown) that in turn couples to a feedback port 135 located on each air zone 160. Second feedback end 324 receives a feedback transfer line 326. Each transfer line 326 couples to a pressure transducer 328 located on the control board 250. pressure transducer 328 receives the pressure from each air zone 160 and transmits to control unit 42 a pressure data signal representing the internal air pressure of the zone 160. Control unit 42 uses these pressure signals to determine the appropriate pressures for certain mattress functions such as CPR, patient transfer, and max-inflate. Pressure signals from the transducer 328 coupled to the foot zone 160k are also used to maintain optimal pressure in foot zone 160k. In the illustrated embodiment, pressure in foot zone 160k (zone 3) is computed as a percentage of the pressure in seat zone 160e (zone 2). The pressures in seat zone 160e and head zone 160f are determined using both the tranducers 328 and the pressure sensors 136. The pressures in one or more of the zones 160 may be adjusted in real time.

As shown in Fig. 23, fill valves 163a-l and vent valves 165a-l are coupled to various portions of patient support 10 through third air lines 150 a, b, d-f, and g-l. Fill valve 163a and vent valve 165a are coupled to upper foot bolsters 140c, fill valve 163b and vent valve 165b are coupled to lower side bolsters 142 a, b, fill valve 163c is coupled to atmosphere and vent valve 165c is reserved for future therapies. Also, fill valve 163d and vent valve 165d are coupled to first turn assist 74a, fill valve 163e and vent valve 165e are coupled to seat bladders 62, fill valve 163f and vent valve 165f are coupled to head bladder assembly 60, fill valve 163g and vent valve 165g are coupled to foot filler 80, fill valve 163h and vent valve 165h are coupled to upper side bolsters 140 a, b, fill valve 163i and vent valve 165i are coupled to deck filler 90, fill valve 163j and vent valve 165j are coupled to first turn assist 74b, fill valve 163k and vent valve 165k are coupled to foot bladders 164, fill valve 1631 and vent valve 1651 are coupled to lower foot bolsters 142c. Vent valves 165d, j are biased in the open position to vent air from first and second turn assist 74a, 74b when first and second turn assist 74a, 74b are not in use. Vent valves 165d, j return to their open position if the mattress loses power or pressure venting air from the first and second turn assist 74a, 74b. When air is vented from a zone 160, the pressure in the zone 160 after deflation is determined by the control system 42, 58 in real time rather than being predetermined.

In one embodiment, a user enters an input command to control unit 42. Control unit 42 processes the input command and transmits a control signal based on the input command through communication line 258 to control board 250. Additionally or alternatively, control signals could be based on operational information from control unit 42 to increase or decrease pressure within one or more of the zones 160 based on information obtained from transducers 328 and/or sensors 136.

It should be noted that in the illustrated embodiment, the mattress controls 42, 58 are independent from operation of the bed flame 4. In other embodiments, however, bed frame 4 and mattress 10 may be configured to exchange or share data through communication lines. For instance, data is communicated from bed frame 4 to mattress system 42, 58 and used to adjust support parameters of mattress 10. For instance, in one embodiment, a signal is transmitted from frame 4 when foot section 34 is retracting, so that mattress systems 42, 58 responds by decreasing internal pressure of vertical bladders 50 in foot assembly 64.

As described above, air supply 152 is capable of supplying air or acting as a vacuum to remove air from zones 160. While in supply mode, a microprocessor on control board 250 actuates corresponding fill valve 163a-l or vent valve 165a-l based on the control signal from control unit 42. For example, if the control signal indicates the pressure in head bladder assembly 160 is to be increased fill valve 163f is actuated. However, if the control signal indicates the pressure in head bladder assembly 160 is to be decreased vent valve 165f is actuated. While in vacuum mode one or more fill valves 163a-l may be actuated to allow for rapid removal of air within the corresponding zones.

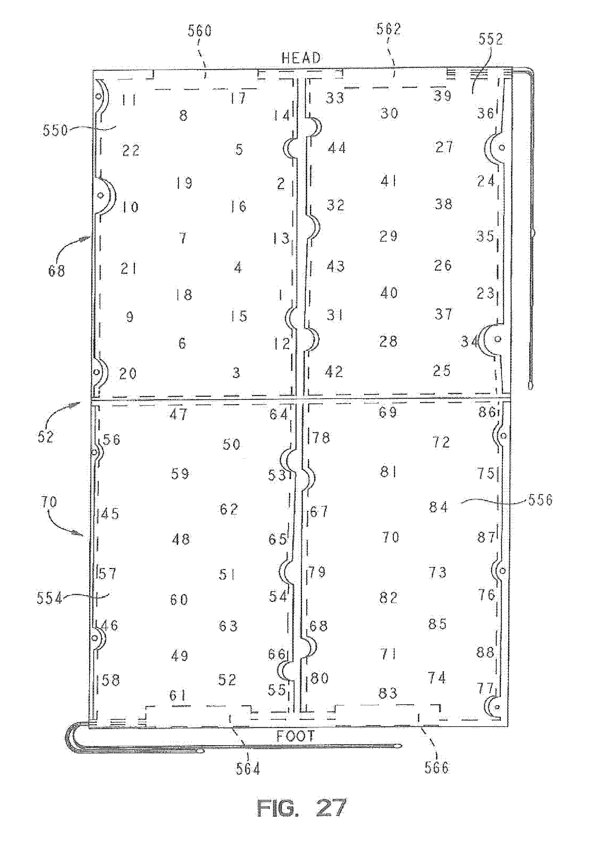

Fig. 27 illustrates the sensor pad 52 including the head sensor pad 68 and the seat sensor pad 70. Each of the pads includes a plurality of sensors configured to provide a reflected wave energy signal is described in PCT Publication WO 2004/00678A1 having a publication date of 22 January 2004, the disclosure of which is incorporated by reference herein. The sensor pads include fiber pairs which introduce wave energy, typically light, into a compressible medium such as foam, The light introduced to the foam is scattered in a manner dependent on the force applied to the surface of the foam. The reflected or scattered light energy is detected and converted to an electrical signal indicative of the force applied the sensor. Both the head sensor pad 68 and seat sensor pad 70 each include 44 individual sensors throughout. The location of each of individual pressure sensing elements is indicated by a number 1 through 88. The sensor pad 68 and the sensor pad 70 each include and can be considered as a collection of 44 independent interface pressure sensors. The areas between sensors are generally not sensitive to pressure. The signals or data generated by the sensors indicate a pressure distribution, the data being essentially a map of the interface pressure between the bottom of the bladder assembly and the deck or frame.

The head sensor pad 68 includes a first sensor group 550 and a second sensor group 552. The first sensor group 550 is located in an upper left quadrant of the sensor pad 52 whereas the second sensor group 552 is in an upper right quadrant of the sensor pad 52. Each of the individual sensor groups 550 and 552 induce 22 sensors, the location of which is indicated and identified by a number. For instance, the first sensor group 550 includes sensors 1 through 22 and the second sensor group 552 includes sensors 23 through 44. The numerical order of the individual sensors indicates the sequence in which the information from each of these sensors is accessed by the multiplexer board 508.

The seat sensor pad 70 includes a third sensor group 554 and a fourth sensor group 556 configured to be substantially the same as the first sensor group 550 and the second sensor group 552 as previously described. Each of the sensor groups includes 22 sensors which have numbers indicating the sequence in which the signal information is accessed or derived therefrom.

Each of the sensor groups 550, 552, 554, and 556 include an optical system device 560, 562, 564, and 566 respectively. Each of these devices include a cable for connection to the pneumatic valve control box 58. Since each of the first sensor group 550, 552, 554, and 556 are substantially identical in construction, the optical system device 560 will be described and its description will apply to the remaining optical system devices 562, 564 and 566.

The optical system device 560 is an opto-electronics interface board including software embedded on a micro controller integrated with an opto-board and the sensor pad itself. The embedded software of the microprocessor is typically referred to as "firmware". As described in PCT publication WO 2004/006768Al, each of the sensors includes fiber optic cable which is coupled to the opto-electric board. Two light emitting diodes supply light to each of the individual sensors and a single photo diode array reads the optical inputs of all 22 sensors within a group. An erasable programmable read only memory and a serial interface driver for communication are included. The primary purpose of the optical system device is to acquire the information sensed by each of the individual sensors which result from the reflected light which has been passed through the fiber optic cable to the individual sensor. Algorithms the embedded microprocessor are used to linearize the data sensed by the sensors. The sensor data and diagnostic data are made available to the multiplexer 508 through RS-232 ports. Data is transmitted though the network 578, which may be a controller area network (CAN) bus, to the algorithm control unit 526.

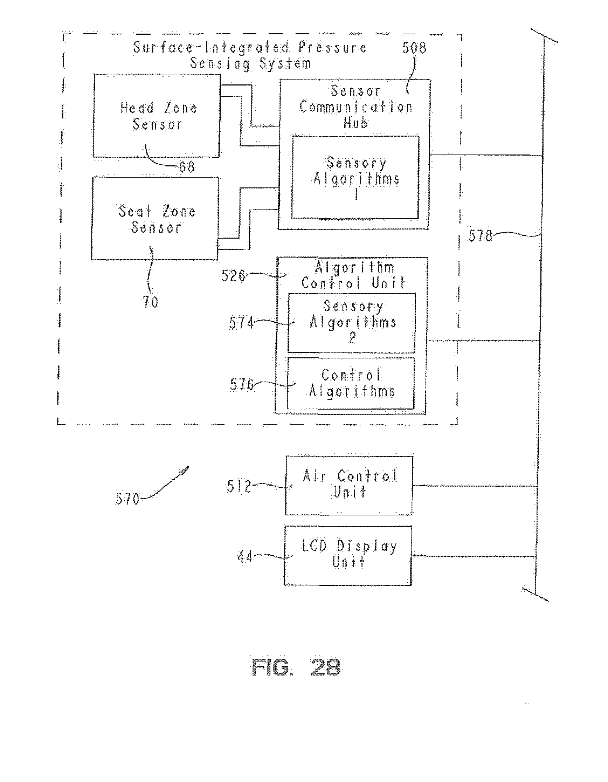

Fig. 28 illustrates an overall system architecture 570 of the present invention, As previously described, the multiplexer board 508, also known as a sensor communication hub, is coupled to the head zone sensor 68 and the seat zone sensor 70. The multiplexer 508 as well as the optical system devices includes a number of sensory algorithms to be described later herein. Also included in the system architecture 570 is the algorithm control unit 526 which includes a second set of sensory algorithms 574 and control algorithms 576. The output of the multiplexer 508 and the algorithm control unit 526 are coupled to a network 578 which is also coupled to the air control unit 512 and the LCD display unit 44. The network 578 includes interface hardware, also known as a communication hub. The network 578 acts as the communication bus for the various hardware, software, and firmware control devices.

As previously described, the multiplexer 508 includes the sensory algorithms 572. The algorithm control unit 526 also includes sensory algorithms which may include algorithms for providing pressure relief, for providing a motion metric, for providing weight estimation, and for providing information to a LCD module which includes a calculation of statistics model.

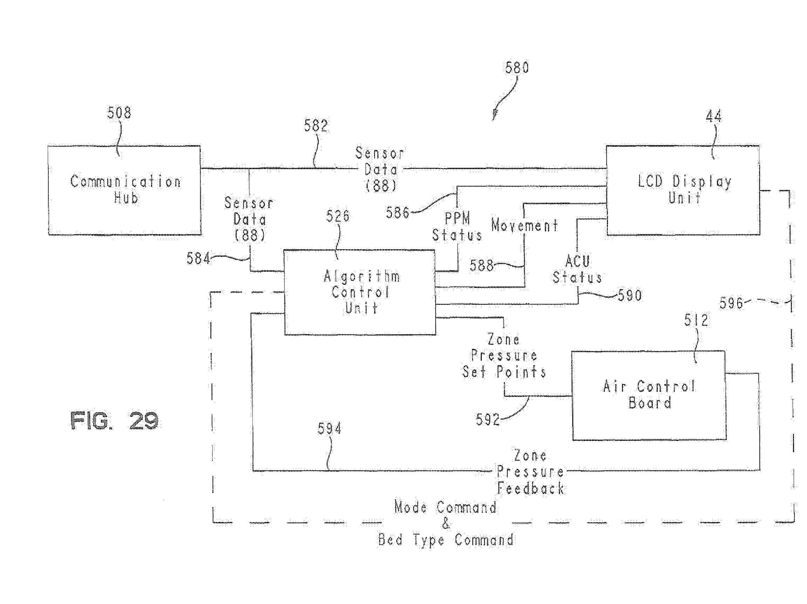

Fig. 29 illustrates a block diagram of a control system 580 incorporating the LCD display unit 44, the air control board 512, the communication hub or network 508, and the algorithm control unit 526. The communication hub 508 which receives sensor data from the head zone sensor 68 and the seat zone sensor 70 is coupled to both the LCD display unit 44 and the algorithm control unit 526 through a first sensor data line 582 and a second sensor data line 584 respectively. As described with respect to Fig. 6, the algorithm control unit 526 includes sensory algorithms 574 and control algorithms 576. The algorithm control unit 526 includes a first output line 586 coupled to the LCD display unit 44 for transmitting patient position monitor statue, a second control line 588 for communicating movement status, and a third control line 590 for communicating the status of the algorithm control unit. In addition, the algorithm control unit 526 includes a fourth output line 592 which transmits the zone pressure set points for each of the head, seat and foot zones to the air control board 512 to which the line 592 is coupled. The air control board 512, which includes the pressure sensors previously described, sends control pressure zone feedbad signals through a line 594 back to the algorithm control unit 526. The LCD display unit 44 through the user input interface device 524 also sends control signals to the algorithm control unit 526 through a control line 596 which includes signals such as various mode command signals as well as bed type command for adjusting the frame or deck of the bed.

As previously described in Fig. 28, the present invention includes sensory algorithms as well as control algorithms. The sensory algorithms are provided in firmware located within the multiplexer 508 and the algorithm control unit 526. Sensory algorithms include the following: bottom out detection, where a portion of the subject is supported by the bed frame as opposed to the surface, bed exit detection, sitting on the side of a bed detection, detection of a patient lying on the edge of the surface, detecting a lack of patient movement on the surface over a period of time, providing patient position monitoring by distinguishing between the following six positions left lying, left sitting, center lying, center sitting, right lying, right sitting, and measuring patient weight within plus or minus 20% within the bed and the flat position. The control system algorithms which are located in the control system algorithm firmware 576 optimize pressure reduction by dynamic load distribution adjustment of the surface air bladders of the mattress 10 located above the head sensor pad 68 and the seats sensor pad 70.

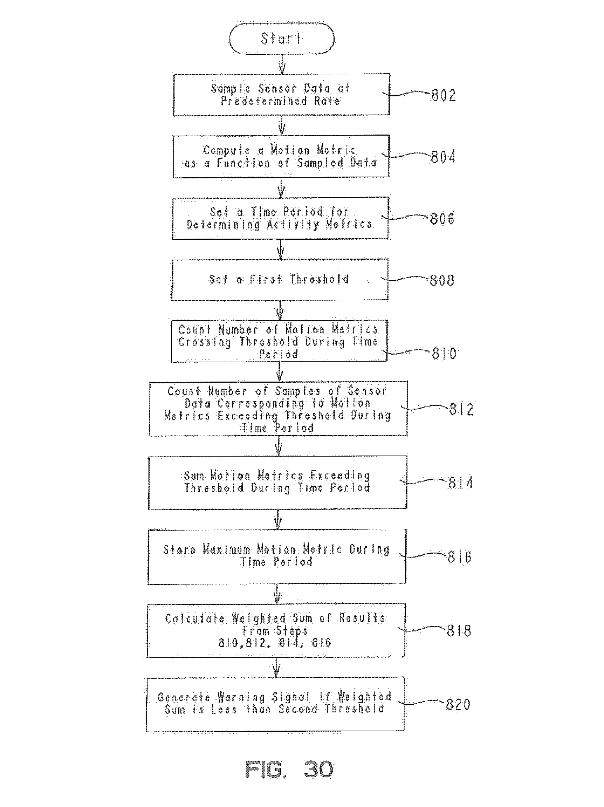

Fig. 30 is a flow diagram for a motion monitor of the present invention. The flow diagram 800 of Fig. 30 provides the steps embodied in the algorithm and firmware of the present invention. To provide patient movement or motion status for use by a caregiver, the sensor data is sampled at a pre-determined rate from each of the plurality of the sensors of the sensor pad 70. The sensors are sampled at a rate of four hertz at stop 802. Due to the large amount of information provided at the sampling rate of four hertz, it has been found that this data received by the algorithm control unit 526 may be transformed into numbers of metrics which provide an accurate indication of patient movement. At step 804, a motion metric is computed as a function of the sampled data. While it is within the scope of the present invention to take a time derivative of the sum of all of the sensor outputs, or to sum the squared time derivative of the individual sensor outputs, or to the absolute value of the time derivative of the individual sensor outputs, the flowchart describes summing the absolute values of the individual high pass filtered sensor outputs so arrive at the motion metric. This motion metric is also called an instantaneous motion metric which may determined according to the following equation:

$$m = \frac{1}{N} \sum_{i=1}^{N} |h(t_i)|$$

$T_1$ is the i-th sensor value where "N" is the number of sensors, and the function H is a (temporal) high pass filter, At step 806, a time period is selected for determining activity metrics at later steps. In addition, a first threshold is set at Step 808 to be described in more detail.

At step 810, a first activity metric is determined in which the number of motion metrics that cross the threshold during the time period is counted. This is called a "zero-crossings" count. At Step 812, a second activity metric is determined in which the number of samples of sensor data corresponding to the motion metrics exceeding the threshold during the set time period is calculated. This activity metric is called "time-over zero". At Step 814, a third activity metric is determined where the amount of the motion metrics which exceed the first threshold during the pre-set time period is summed together. At Step 816, a fourth motion metric is determined where the maximum motion metric which occurs during the time period is stored. Each of the activity metrics determined at Steps 810, 812, 814, and 816 can be represented as a single value which occurs during the set time period established at Step 806. Each of these activity metrics are then combined into a single value at Step 818 by computing a weighted sum of the four activity metrics. Coefficients used in the weighted sum are selected in a manner that differentiates a patient's motion of interest. In this manner, the magnitude of movement of a patient over the pre-determined time period may be compressed into a single value. Once this value is determined at Step 818, it may be compared to a second threshold. If the weighted sum is less than the second threshold, then a warning signal may be transmitted over the line 588 to the LCD Display Unit 44.

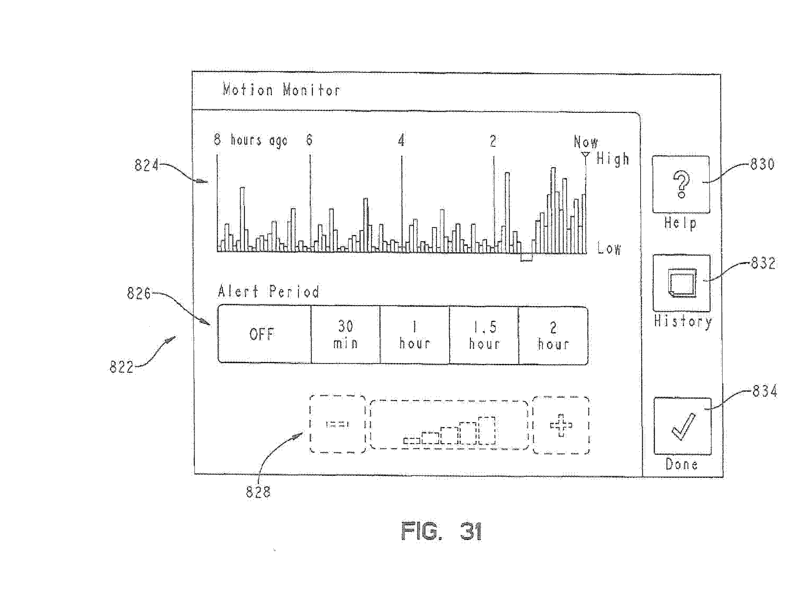

Fig. 31 is a screen display of a motion monitor user interface screen of the present invention. The interface screen 822 includes a first display portion to display the weighted sums in a graphical user interface. A second display portion 826 includes a number of user interface buttons for adjusting the time period over which a patient's motion is monitored. These buttons may include a touch screen display or other user input devices, As illustrated, the monitored time periods may be selected as 30 minutes, 1 hour, 1.5 hours and 2 hours, as well as an off position. A third display portion 828, here shown in dotted outline, enables a user or a caregiver to adjust the second threshold level as described in Step 820 of Fig. 30. The third display portion may be displayed as a lighter shade or as a background image when the monitor is in the off position. In addition, the user interface 822 includes a help button 830 to provide instructions for a user or caregiver, a history button 832 to be described later, and a or complete button in which the caregiver can move to a different set of user interface screens.

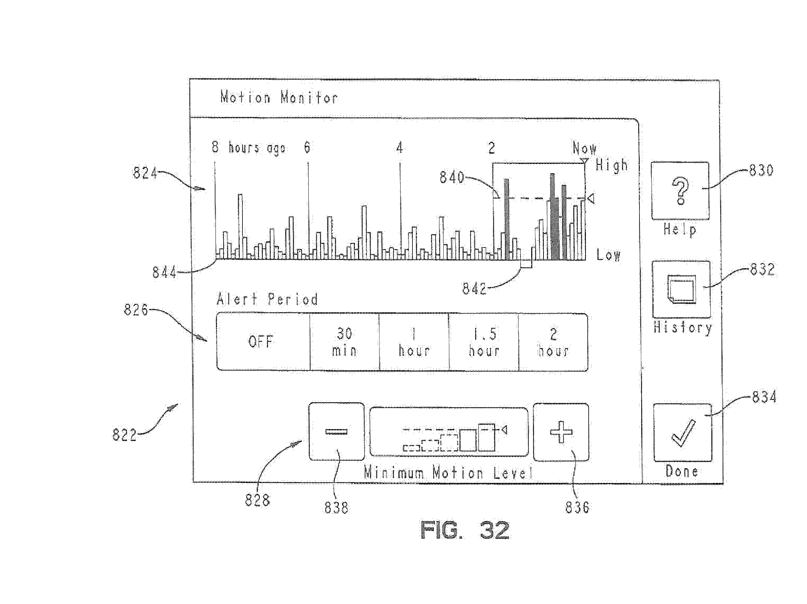

As illustrated in Fig. 32, the caregiver may select from of five threshold levels in the third display portion 828. When an alert period is selected, the third display portion 828 is visually enhanced to indicate an alert period has been selected. The selected threshold level, also shown here as a minimum motion level, establishes a minimum activity level which the caregiver selects to monitor the patient's activity level. As shown, a plus button 836 allows the caregiver to increase the second threshold or minimum motion level and a minus button 838 allows the caregiver to reduce the second threshold or minimum motion level. If the activity level of the patient stays below the set threshold the selected alarm period, an audible and a visual alarm may activate or sound. A bar chart in the first display portion 824 is updated every 5 minutes. This 5 minutes of activity corresponds to the time period selected for determining activity metrics as determines in Step 806 of Fig. 30. Other time periods are within the scope of the present invention, Each bar of the portion 824 is shown to be 5 minutes wide.

Each of the vertical bars of the first display portion 824 corresponds to one of the five minute time periods, A threshold level line 840 is displayed to indicate the minimum motion level set by the buttons 836 or 838. When it is determined that the minimum motion level exceeds the threshold 840, the bars of the bar chart are highlighted to indicate that the minimum motion level has been exceeded, While the bars of the bar chart exceeding the level 840 are illustrated as shaded bars, it is within the scope of the present invention to shade the bars with different levels of gray or to color code the individual bars for use with a color display. The bar chart also indicates when the bed is empty with a bar 842 which extends below a low line 844. Bed empty is determined by the sensors having sensed a lack of patient weight or force.

Every 30 minutes, a calculation is made to determine if any of the 5 minute bars have surpassed the caregiver selected minimum motion level. While these threshold calculations are performed every 30 minutes, an alarm is only activated at the end of the caregiver selected alert period as illustrated at the second portion 826. For example, if a period of 2 hours (120 minutes) is selected as the time period, calculations may be made before an alarm occurs at the end of the 2 hour time period. If each of the four calculations, one for each 30 minute time period, determines that the threshold has not been surpassed, an alarm will sound. However, if the threshold has been surpassed during any of the four periods, then no alarm will sound and the motion monitor will continue to record and calculate data. Correspondingly, the 1 hour and 1.5 hour alarm periods require respectively two and three consecutive 30 minute calculations with the threshold not being exceeded to cause an alarm.

If the threshold is not exceeded as described, a visual and audible alarm signals to the caregiver that the patient should be attended to. The caregiver attending to the patient cancels the alarm. The pre-set time period will then start again. During this new period, patient activity will continue to be monitored and displayed on the screen. If the threshold has been surpassed, indicating that the patient is moving, then the motion monitor continues to acquire data in 5 minute increments and threshold calculations at the end of each 30 minute period are performed to determine an alarm condition.

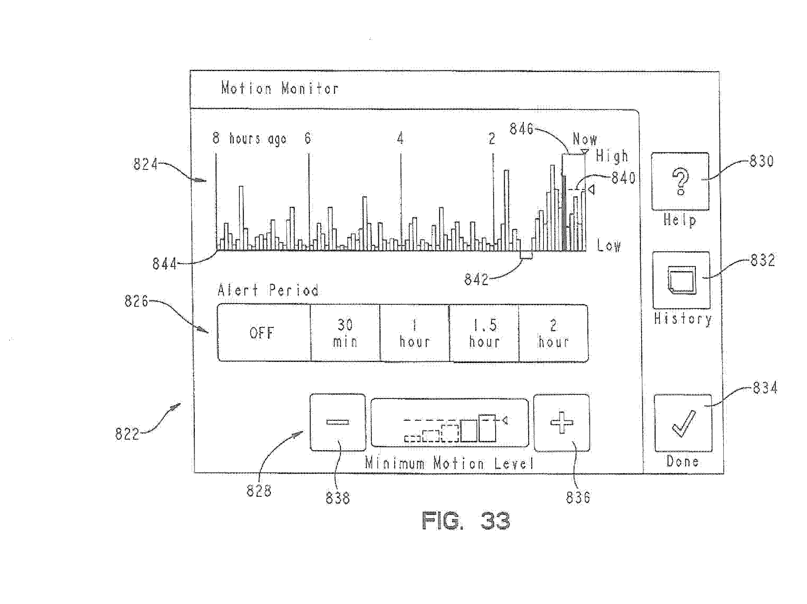

Fig. 33 is a screen display 822 of the motion monitor user interface screen when the motion monitor is set to a time period of 30 minutes. As can be seen in Fig. 11, the caregiver would adjust the alert period by pressing the 30 minute button such that the bar chart or graphical display located in the first portion 824 indicates that a monitoring period of 30 minutes has been selected. The monitoring period of 30 minutes is defined by the user interface to include a rectangular portion 846 which encompasses or surrounds the bars within the 30 minute time period. This section, while indicated with lines, may also be indicated by using background colors such that the background color of the 6 bars making up the 30 minute time period may be displayed with a background of a different color than the bars. This rectangular portion may also be seen in Fig. 32. As before, if any of the individual bars within the 30 minute time period exceed the set motion level, the bars are indicated as a shaded bar.

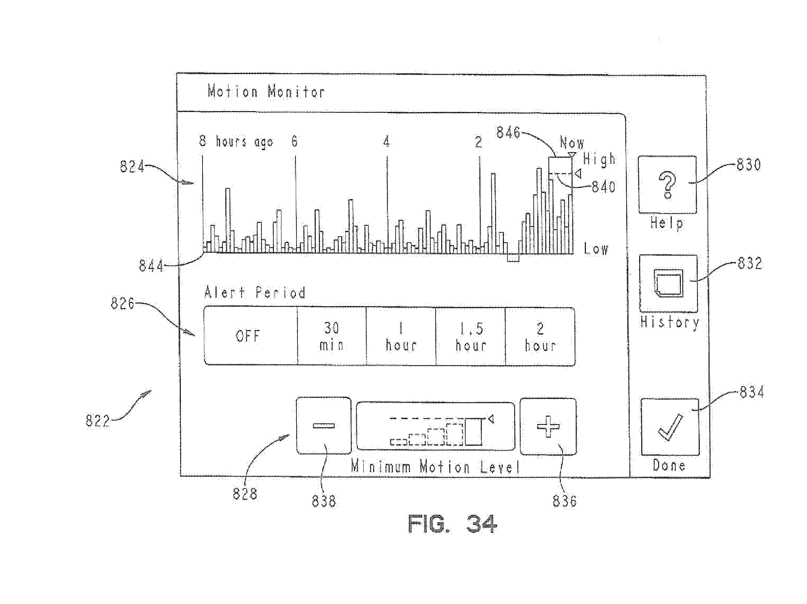

Fig. 34 is a screen display of the motion monitor user interface when the motion monitor time period is set to 30 minutes and the minimum motion level is set to the highest setting. In this illustration, none of the individual bars exceeds the set threshold level 840 during the 30 minute time period. Consequently, none of the bars have been highlighted to indicate that the threshold level has been exceeded. In this situation where none of the bars have exceeded the minimum set motion level, an alarm would sound to indicate to the caregiver that the minimum motion level has not been reached.

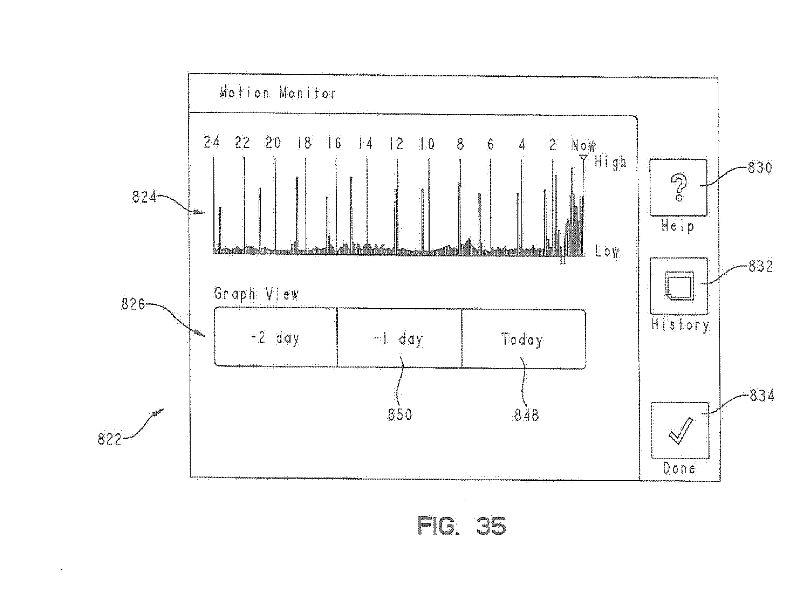

Fig. 35 is a screen display of the motion monitor user interface screen 822 when the history button 832 is selected to display a default history view showing the previous 24 hours of monitored motion. Other periods of default history, such as the previous 8 hours may also be selected and displayed. To view the history profile, which is displayed in the first section 824, the caregiver presses the history button 832 upon which the bar chart of the section 824 is displayed, Since the default condition is a representation of the motion for the last 24 hours, a 24 hour bar chart is displayed with each of the individual bars for the entire time period being displayed. In addition to the amount of the patient's activity level, the time patient activity occurs during the day can also be tracked. This information may be used by a caregiver to make care decisions for the patient. In addition to displaying a period of the previous 24 hours by pushing a Today button 848, the previous days activity level may be viewed by pressing a minus one day button 850, Pressing the minus one day button displays a period of 24 hours beginning 24 hours prior to the current time and ending 48 hours prior. Likewise, pushing the minus 2 day button displays a time period beginning 48 hours prior to the current time and extending for 24 hours prior to that start time.

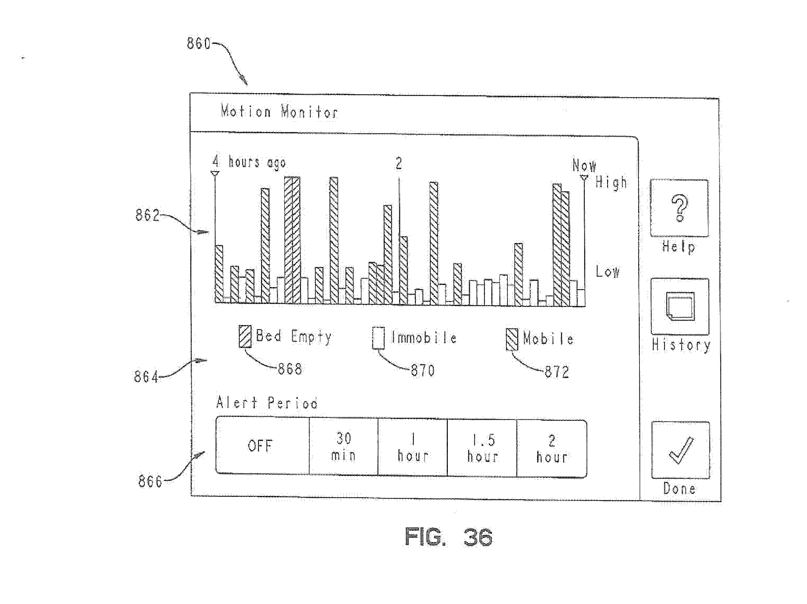

Fig. 36 illustrates another embodiment of a screen display 860 of the present invention. In this embodiment, a first display portion 862 includes a bar grape, A second display portion 864 defines a state of the patient or bed corresponding to each of the bars of the first display portion 862. These states include bed empty, immobile, and mobile. A third display portion 866 enables a caregiver to select the alert period over which patient movement is monitored to provide an alarm. For the embodiment of Fig. 36, the width of each of the bars displayed in part in 862 is 5 minutes. In this embodiment, the interface does not provide for the election a threshold level. Instead the threshold level is set internally in the firmware residing in the algorithm control unit 526.

In Fig. 36, the alert period has been turned off, Consequently, while it is possible to see patient movement status which includes a bed empty status 868, a patient immobile status 870, and a mobile status 872, the system is not placed into an alarm mode where an alarm is provided as previously described. In this mode, the system automatically graphs patient motion, immobility, as well as out of bed or bed empty status in a 4 hour rolling graph. The caregiver may access the graph by pressing the motion monitor button which is present in a menu. In the case of immobility, the Braden scale has been used where immobile is defined as breathing, wiggling extremities, and turning the head side to side.

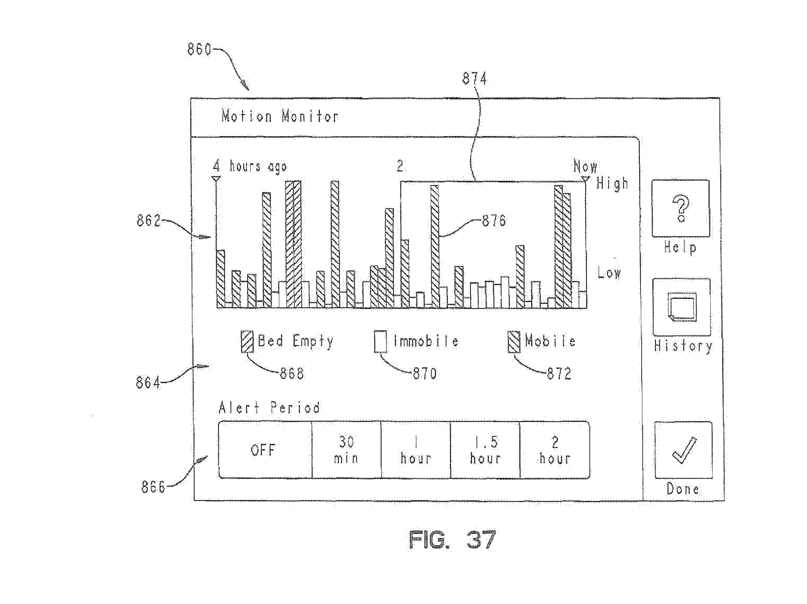

Referring now to Fig. 37, the caregiver has selected a 2 hour time period which establishes an alarm period for determining whether or not a patient is immobile during that time. The user interface 860 indicates that a 2 hour time period has been selected by displaying a rectangle 874 from the current time to a time of 2 hours previous. The two hour time period includes a plurality of mobile states 876, thereby indicating that the patient has sufficient mobility. In this instance, an alarm is not sounded.

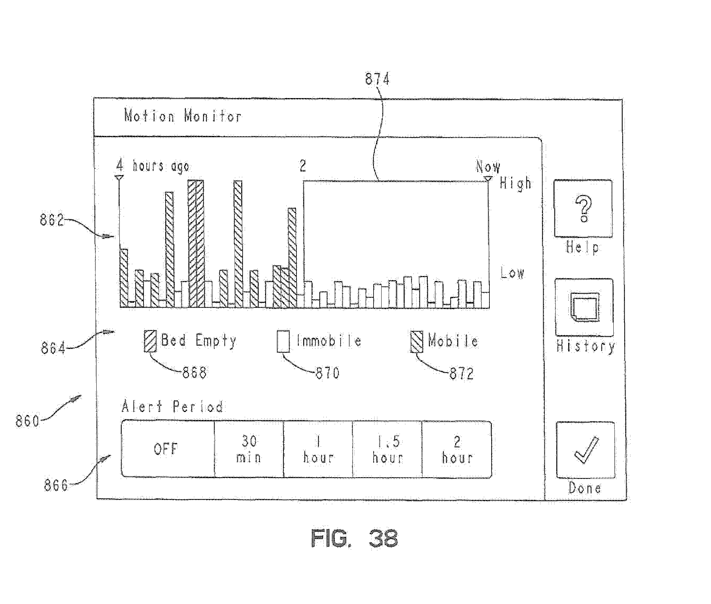

Referring now to Fig. 38, a screen display 860 is shown where a patient has been determined to be immobile for a selected 2 hour period. Only the immobile bars 870 are displayed over the 2 hour period 874. Consequently, the patient has remained immobile for the entire time, Since none of the mobile states 872 have occurred during the 2 hour window, an alarm would sound.

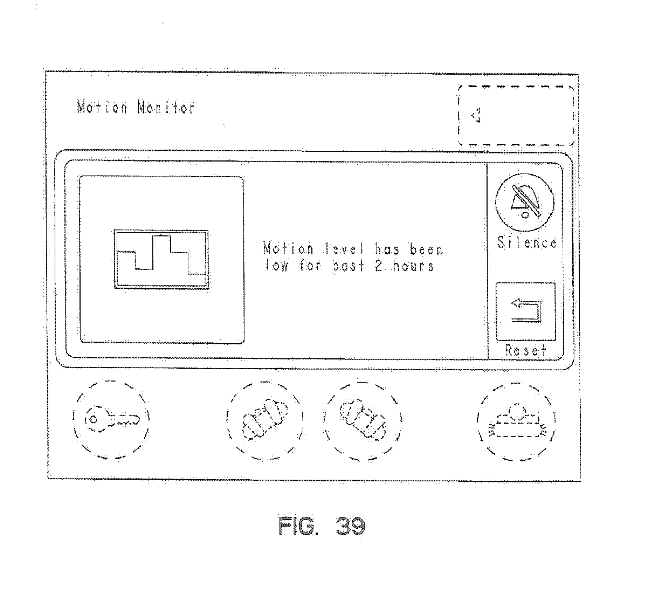

An audible alarm may be made with the speaker 530 of Fig. 24B. In addition, as illustrated in Fig. 39 a visual alarm is made on the user interface. A warning is displayed which states that the motion level has been low for the past 2 hour. A similar type of display may be used for those instances where a threshold level has been set such as described with respect to Fig. 34. In the embodiment, the warning may state that the motion level is under the threshold setting during the past 90 minutes if a 90 minute period has been selected.

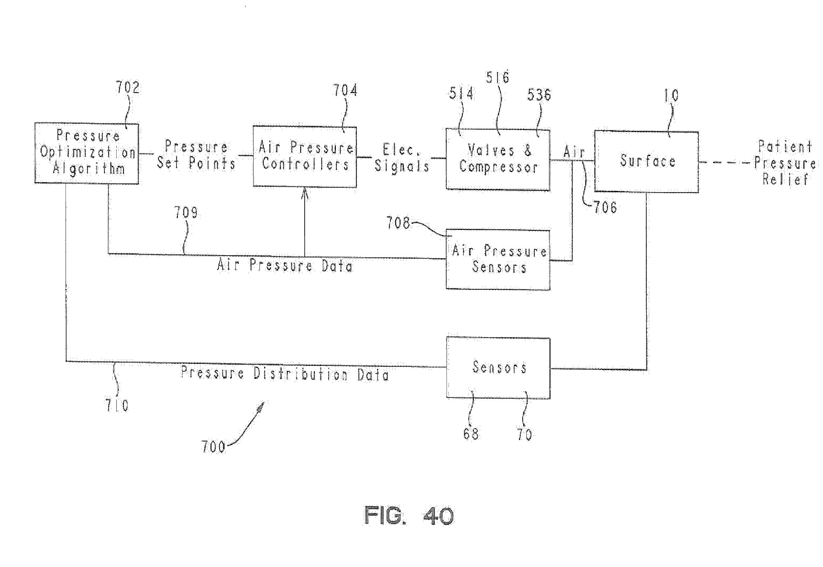

Fig. 40 illustrates a block diagram for a pressure optimization control system 780 of the present invention. The control system provides closed loop feedback to find a preferred air pressure for supporting a patient. The air pressure located within the bladders located above the head sensor pad 68 and the seat sensor pad 70 is adjusted according to pressures sensed by pads 68 and 70. The patient to mattress interface pressure is not measured directly. Instead, the sensors located within the head sensor pad 68 and the seat sensor pad 70 sense the force or pressure transmitted through the associated head and seat bladder sections, The air pressure within each of these sections is measured by a pressure transducer located within the pneumatic valve control box 58.

As illustrated in Fig. 40, a pressure optimization algorithm 702, located within the control algorithm firmware section 576, transmits pressure set points stored or generated by the algorithm 702 to air pressure controllers located within the air control board 512. The air pressure controllers 704 generate control signals which are transmitted and coupled to the valves located within the first valve block 514, the second valve block 516, and to the compressor 536 located within the control unit 42, The controlled flow of air is provided to the surface 10 for pressure relief of the patient. The air within the air line 706 is coupled to and monitored by air pressure sensors 708 which transmit pressure signals through a line 709 to both the air pressure controller 704 and to the pressure optimization algorithm 702. In addition, the head sensor pad 68 and seat sensor pad 70 also measure the force supplied through the surface 10 and force sensing information is transmitted back to the pressure optimization algorithm 702 along a line 710.

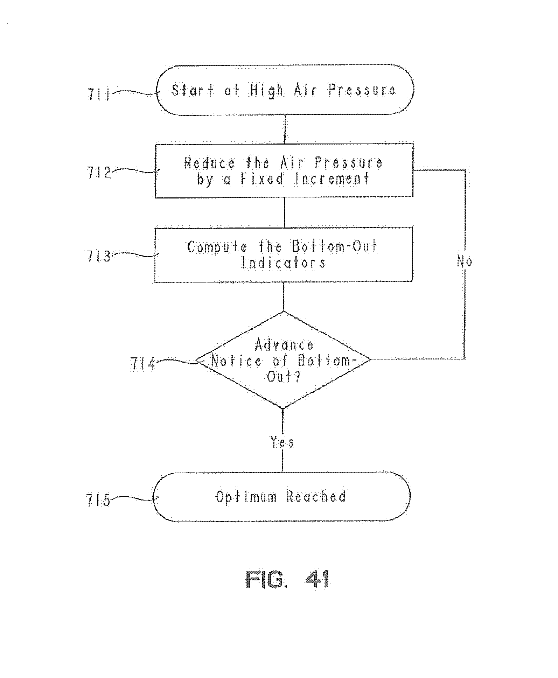

Fig. 41 is a flowchart illustrating a method of determining a preferred pressure for the patient support of the present invention. The present invention provides for pressure relief within the air bladders by monitoring the air pressure within the bladders and controlling that air pressure through the detection of the force of pressure transmitted through the air bladders to the sensors located therebeneath.

Based on the assumption that the optimum air pressure is the pressure just prior to bottoming-out, this indication may be used as advance notification or as a signal that the optimum pressure has been reached. As shown in Fig. 41, the air pressure is reduced in increments. After each increment, the bottoming-out indicators are computed. At such time as the indicators provide notice of the bottoming-out trend, the air pressure is maintained at that setting, and the optimum pressure relief has been achieved. In principle, this algorithm may be used to automatically determine the optimum air pressure for different individuals and for different postures on the bed.

When the system is first turned on, the controller adjusts the air bladder pressures to a high air pressure at block 711. Initially, the bladders may be filled to 25 inches of water. The patient is then placed on the mattress where the mass of the patient is calculated according to a mass or weight algorithm. Once the mass of the patient has been calculated, the pressure within the bladders is lowered by fixed increments at block 712 of Fig. 41. As the pressure is lowered, the sensors of the sensor pads 68 and 70 are accessed according to the sequences previously shown in Fig. 27. The data or information provided by 22 of the sensors within each of the sensor groups is read or provided approximately every one quarter of a second. Consequently, the information from all of the first, second, third, and fourth sensor groups 550, 552, 554, and 556 are provided approximately every one second. This information is used to compute bottom-out indicators at block 714. The bottom-out indicators are derived from the pressure distribution data derived from the sensors from each of the sensor pads 68 and 70.

The bottom-out indicators are used to determine a bottoming-out trend. Such indicators may include.

(a) The sum of outputs of sensors over a "high pressure threshold." For this indicator, a threshold is set, and the amount by which the sensors exceed this threshold is accumulated. The high-pressure threshold may be fixed, or preferably, it may be computed from time to time in proportion to the average sensor output. It has been found that it is preferable to set the high-pressure threshold in the range of 1.2 to 3.0 times the average of all sensor outputs.

(b) The area not providing support, as measured by the number of sensors below a "support threshold". The "area not providing support" decreases when the support area increases. The support threshold may be fixed, or preferably, the support threshold may be computed from time to time in proportion to the average sensor output. It has been found that it is preferable to set the high-pressure threshold in the range of 0.1 to 0.7 times the average of all sensor outputs.

(c) The number of sensors over a high-pressure threshold. Similar to the indicator described in (a) above, a high-pressure threshold is set, and the number of sensors that exceed that high-pressure threshold is counted.

(d) The maximum output reported by any given sensor.

(e) The average of the three sensors reporting the highest outputs.

(f) The standard deviation of all of the sensor outputs. This is calculated in accordance with the formula: standard deviation equals the square root of the sum of squared differences between the sensor output and the mean sensor output, divided by the number sensors minus one.

(g) The high-side deviation of sensor outputs. This indicator calculated in a similar manner to the standard deviation. In this case, however, only those sensor outputs that exceed the mean sensor output are used in the computation.

(h) The changes in the above indicators as a ratio to the change in bladder air pressure.

The pressure optimization algorithm 702 determines a distributed standard deviation of the data to provide an indicator which corresponds to a pressure within each of the head and seat bladder sections, As the distributed standard deviation trends toward a certain value, the air pressure is reduced continually at block 712 as long as the advance notice of bottoming-out at decision made at block 726 is not indicated. If, however, the advance notice of bottoming-out does occur as determined at decision block 714, then the preferred or optimum value of pressure is reached at block 715. The pressure optimization algorithm 702 then sends signal to the air pressure controller 704 to maintain the pressure within the head and seat zones. The pressure or force transmitted through the head and seat zone bladders is continuously monitored and used to adjust the pressure within the bladders.

While the algorithm 702 reduces the air pressure by fixed increments at block 712 of Fig. 41, the firmware includes a reference table of patient weights and corresponding pressures. Initially, the bladder pressure of 25 inches of water is not incrementally dropped to a lower level. Instead, it is dropped by a larger amount, for instance 8 inches of water, to thereby reduce the time it takes for the system to reach the patient's optimized pressure profile. Since the table includes pressures correlated to patient weight, the system achieves an optimum state more quickly than if the pressure was reduced by fixed increments from the initially set pressure of 25 inches of water.

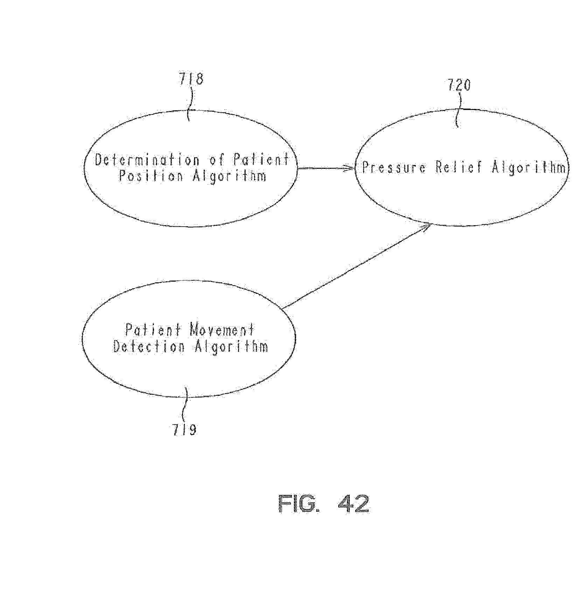

The flowchart with respect to Fig. 41, which determines a preferred pressure for the patient support of the present invention, may include a first algorithm 718 of Fig. 42 which determines the patient position and a second algorithm 719 which determines a patient movement or patient movement detection. The determination of patient position algorithm 718 determines the location of a patient by using the sensors 52 as previously described with respect to Fig. 27. Outputs from each of the individual sensors can be used to determine the location of the patient. The following patient locations or patient states are determined: Bed empty, centerline, left side lying, right side lying, left side sitting, right side sitting, and center sitting. In addition to the determination of the patient position, the patient movement detection algorithm 719 quantifies the amount of movement of a patient on the surface. This movement is quantified by monitoring the pressure changes which occur with respect to the bladders but which is sensed with the sensors 52. The output of the patient position algorithm 718 and the output of the movement detection algorithm 719 are used by a pressure relief algorithm 720. As generally described with respect to Fig. 41, the pressure relief algorithm reduces air pressure by fixed increments where the bottom-out indicators are used to detect advance notice of bottoming-out.

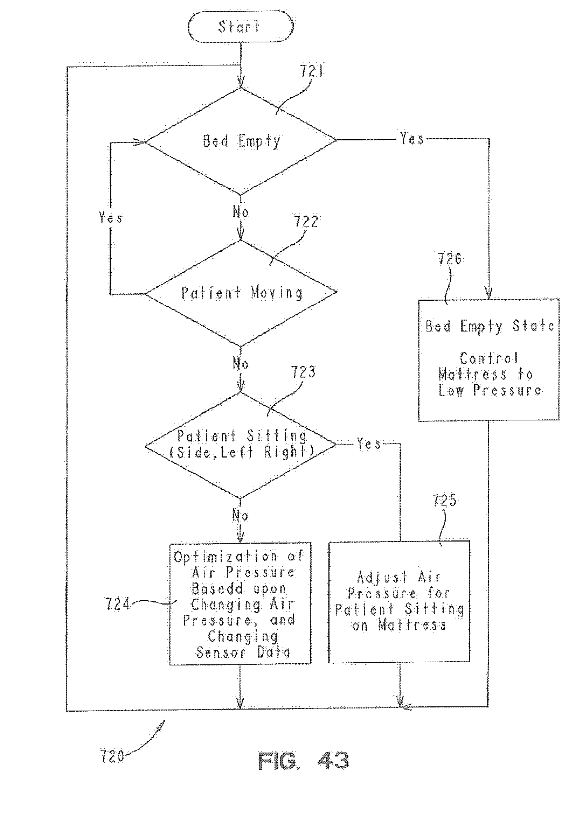

Fig. 43 illustrates a flowchart of a method of optimizing air pressure for a patient. Initially the pressure relief algorithm 720 determines at a decision point 721 whether or not the bed is empty. If the bed is empty, the algorithm moves to a bed empty state 726 where the mattress pressure is set to a lower pressure singe a patient is not located on the mattress. If, however, it is determined at step 721 that the bed is not empty, then the algorithm at step 722 determines whether or not the patient is moving. If the patient is moving, then the patient moving step 722 is repeated until it is determined that the patient is not moving. If the patient is not moving, the pressure relief algorithm 720 utilizes the patient position information which has been determined by the patient position algorithm 718. If the patient is sitting either on a left or right side at step 723 then the air pressure within the seat section is adjusted at step 725 for the patient sifting on the mattress, If, however, it is determined at step 723 that the patient is not in a sitting position the algorithm optimizes the air pressure. Air pressure is optimized and is based upon changing air pressures and changing sensor data to be described herein.

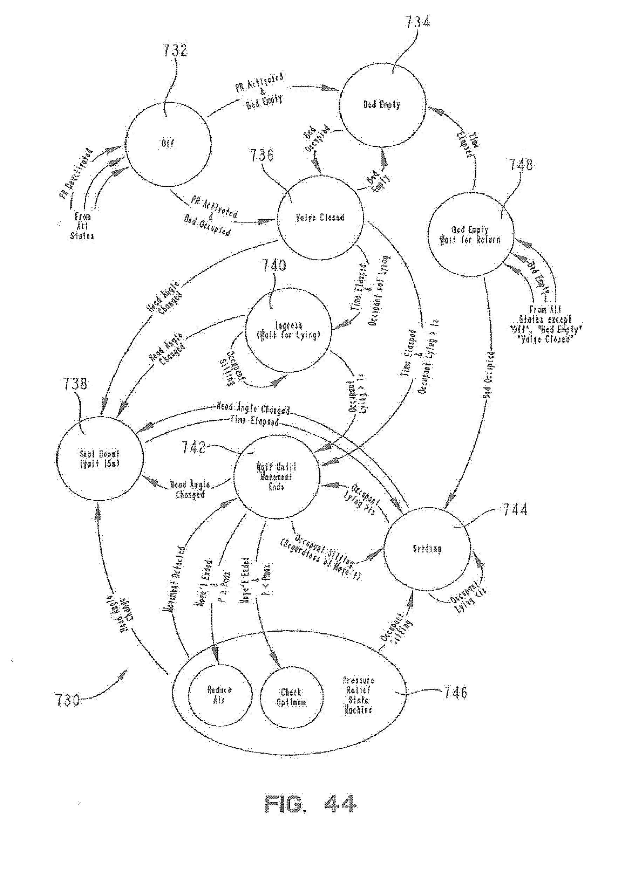

Fig. 44 illustrates a state machine diagram for the control system of the present invention. The state machine diagram 730 indicates the various states of the present system which are enabled by firmware. The state machine represents the behavior of the present mattress system which is dependent upon the outcome of the various algorithms as well as the calculation of a number of indicators. The state machine diagram indicates the behavior of the system made in response to sensed conditions or derived values. These conditions and values include (1) indicators, (2) the patient position monitor, and (3) air pressure. In the figure, the curved arrows indicate the allowable transitions between states. The conditions that precipitate a transition from one state to another are labeled on each arrow.

Indicators are derived from the sensor outputs, For instance, the current state of each of these sensors is accessed over a period of in the sequence previously described in Fig. 27. When that information is sensed over a period off time, the stored information may be used to develop an indicator. For instance, one of the indicators includes determining the standard deviation over the average, Indicators are calculated using the sensor outputs over a period of time and stored in memory. A change to the indicator may be compared to a predetermined threshold value or to other values which are based on the indicator values themselves, For instance, if the indicators are derived over a period of time, it is possible to determine the minimum indicator value during that period of time. The minimum indicator may then be used to calculate a threshold value equal to a percentage of that minimum value. That calculated value may then be compared to indicators to change from one state to another state.

In the present invention, the bottoming-out indicators, the standard deviation divided by the average over time, are used to provide advance notice of bottoming-out. An assumption is made that the optimum air pressure for an individual patient its at a pressure point just prior to bottoming-out. The indicators may then be used as an indication of the pressure within the bladder and whether it is increasing or decreasing. Consequently, bottoming-out can be predicted. Pressure is adjusted based on the predicted bottoming-out. Once the optimum pressure has been reached the pressure may be continually adjusted to maintain that pressure.

As previously described, after each increment or decrement of pressure, the bottoming-out indicators may be re-computed. Once the bottoming-out indicators provide advance notice of bottoming-out, then the air pressure is maintained at that setting and the optimum or preferred pressure relief is achieved. Such an algorithm provides a method to determine the optimum air pressure for a variety of individual patients and for different postures on the bed.

Referring now to Fig. 44, a state transition diagram for the patient support system is disclosed. Each one of the states represents a corresponding software function that may be embodied as software or firmware. Starting with an off state 732, the off state 732 may be entered from all of the states as well as when the pressure reduction (PR) is deactivated. (Pressure reduction may be deactivated by a user through the user interface 44.) A transition to a bed empty state 734 may be made by activating the pressure reduction as well as when the bed is empty. Once in the bed empty state 734, that state can be changed if it is determined that the bed is occupied. If it is determined that the bed is occupied while in the bed empty state 734, then the valve closed state 736 is entered. In addition, the valve closed state 736 may be entered from the off state when pressure reduction is activated and the bed is occupied.

In the valve closed state, the valves are closed for a set period of time while the system determines whether or not the occupant or patient or the frame itself transitions through a change of state. If while in the valve closed state 736, it is determined that the bed is empty, then the state transition diagram returns to the bed empty state at 734. If, however, if in the valve closed state 736, it is determined that the head angle has been changed, then the system moves to a seat boost state 738. In the seat boost state 738, the pressure in the seat is increased or boost for approximately 15 seconds, If, however, while in the valve closed state 736, the head angle is not changed but a certain period of time elapses and it is found that the occupant is not lying, then the system moves to the ingress state 740 which indicates that a patient is entering the bed. While in the ingress state 740 the system waits to determine whether or not the patient is in a lying position. If it is determined while in the ingress state 740 that the occupant is in a sitting position, then the state diagram maintains the ingress state 740.

Transition from the ingress state occurs if it is determined that the occupant or patient is lying for a period of greater than one second. The wait until the movement ends state 742 is entered. If while in the wait until the movement ends state 742, the head angle of the deck is changed, the system enters the seat boost state 738. If, however, it is determined that the occupant is sitting while in the state 742, the system moves to a sitting state 744. While the system determines that a patient is in a sitting position at state 744, as long as the patient or occupant is lying for less than one second, then the system remains in the sitting state 744. If while in the sitting state 744 the head angle is changed, the system moves to the seat boost state 738 in which the seat bladder is boosted for approximately 15 seconds, After that time has elapsed in the state 738, the system returns to the sitting state 744.

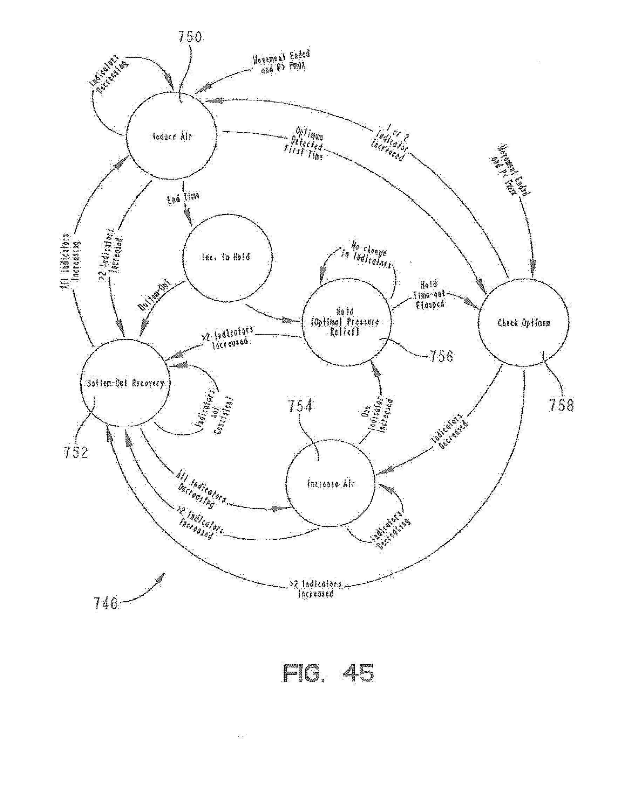

If while in the sitting state 744, it is determined that the occupant is lying for greater than one second, then the system transitions to the wait until the movement ends state 742. If the system determines that the movement has ended at state 742, then the system moves to the pressure relief state 746 under two conditions. Those two conditions are: 1) when the movement has ended and the pressure is greater than the maximum pressure or 2) when the movement has ended and the pressure is less than the force maximum. During the pressure relief state 746, pressure is adjusted for a patient in the prone position to be described in greater detail in Fig. 45. If the occupant, however, sits up during this state, then the system moves from the state 746 and returns to the sitting state 744. Likewise, if the head angle is changed while in the pressure relief state 746, then the system moves to seat boost state 738. The system can also leave the pressure relief state 746 when movement is detected, The detection of movement indicates that pressure relief is temporarily stopped until the movement ends at which point the system returns to the pressure relief state where the air pressure is continuously monitored and adjusted when necessary to provide optimum pressure relief. A bed empty wait for return state 748 may be entered when the patient leaves the bed. The bed empty wait for return state 748 may be entered from all states except the off state, the bed empty state 734, and the valve closed state 736.

Fig. 45 illustrates a state transition diagram for the pressure relief state machine 746. As previously described, the bottoming-out indicators provide advance notice of bottoming-out. Based on the assumption that the optimum air pressure is the pressure just prior to bottoming-out, this advance notification is used as a signal that the optimum or preferred pressure has been reached, As previously described, air pressure is reduced in increments. After each increment the bottoming-out indicators may be computed, At the time that the bottoming-out indicators provide advance notice, then the air pressure maintained is that at that setting and the optimum or preferred pressure relief is achieved.

In the figure, the curved arrows indicate the allowable transitions between states. The conditions that precipitate a transition from one state to another are labeled on each arrow. In some cases, the reasons are based on a count of the number of indicators meeting a certain condition (eg. ">2 indicators decreasing"), It is to be understood that conditions may be replaced by comparing a single indicator (or weighted sum of indicators) against a suitable threshold, If it is determined that the movement has ended and that P is greater than or equal to P max, then the air is reduced at a reduce air state 750. If it is determined that the indicators are decreasing, the system continues to reduce the air in the mattress bladders. If, however, it is determined that more than two indicators are increasing, the system enters a bottoming-out recovery state 752. The system remains in the bottoming-out recovery state if the indicators are not consistent. If, however, the indicators are increasing, then the system returns to the reduce air state 750. If, on the other hand, all indicators are decreasing, then the system enters an increase air state 754 where the air within the bladder is increased. The system remains in the increase air state 754 if all indicators are decreasing.

If more than two indicators increase, the system leaves the increase air state 754 and returns to the bottoming-out recovery state 752. If one indicator increases, then the system moves to the hold state 756 where the air pressure within then mattresses is maintained for the optimum or preferred pressure relief. If there are no changes to the indicators while in the hold state 756, the system remains in the optimal pressure mode. If, however, more than two indicators have increased while in the hold state 756, the system returns to the bottoming-out recovery state 752 as previously described. While in the hold state 756, a timer is set which enables the system to check for an optimum state at check optimum state 758 after the time out has elapsed. When in the check optimum state 758, if one or two indicators have increased, the system returns to the reduce air state 750 where the air in the bladders is reduced. If the optimum state is detected while in the reduced air state 750, the system moves to the check optimum state 758. A timer may also be set while in the reduce air state 750 whereupon at the end of the elapsed time the system returns to the hold pressure state 756. If during the transition 746 movement is detected the systems returns to the wait until movement ends stage 742 of Fig. 44.

When the bed is empty, the automatic control system is in the "Bed Empty" state. In this state, the control system sets the air pressure set-point to a value sufficient to fully inflate the air bladder.

It is known how to determine whether a patient has entered the bed (see for example, Lokhorst et al PCT international Publication WO 2004/006768) using an interface pressure sensor. Alternatively, other means, such as load cells in the legs of the bed frame or capacitive sensors or other types of bed occupant detection switches, may be employed to determine if a person occupies the bed, As soon as an occupant is detected, the automatic control system switches into the "valves closed" state. In this state, the automatic control system transmits instructions to the air pressure regulator to close off airflow in and out of the air bladder (essentially, to stop regulating the air pressure for the time being). When a fixed time period has elapsed, preferably about 5 to 30 seconds, the automatic control system switches into the "reduce air" state.

In the "reduce air" state, the automatic control system instructs the air regulator to reduce the air pressure by some increment. After a period of time, the indicators are computed. If the indicators have reduced, then the automatic control system remains in the "reduce air" state and initiates another decrement to the air pressure. If an indicator or two are found to have increased, then it means that the bottoming-out trend has started, and so the automatic control system switches to the "hold" state.

In the "hold" state, the automatic control system instructs the air regulator to maintain the air pressure at the value it was when the state was entered. Periodically, the indicators are computed. If there is no significant change in indicators, then the automatic control system remains in the "hold" state. If an indicator increases while in the "hold" state, it may be indicative of the occupant moving. In that case it is necessary to conduct a test to determine if the air pressure presently being maintained is optimal. This test is automatically conducted by switching to the "check optimum" state.

In the "check optimum" state, the automatic control system instructs the air pressure regulator to increment the air pressure by some interval. When the desired increase in air pressure has been achieved (or, alternatively, a reasonable length of time has elapsed), the indicators are computed. If the indicators decreased, it indicates that another increment in air pressure is required, so the system switches so the "increase air" state (which is subsequently described). As previously stated, the indicators were chosen so that minimum values are reached at or about the lowest air pressure prior to bottoming-out. Therefore, if the indicators decrease with increasing air pressure, then it indicates that the air pressure is still too low - further increasing the air pressure is likely to further reduce the indicators. If, on the other hand, the indicators generally increase after the increment in air pressure, then the opposite is true: the air pressure is now higher than optimum, and the system switches into the "reduce air" state.

In the "increase air" state, the automatic control system instructs the air regulator to increase the air pressure by some increment. After a period of time, the indicators are computed. If the indicators have reduced, then the automatic control system remains in the "increase air" state and initiates another increment to the air pressure. If an indicator or two are found to have increased, then it means that the bottoming-out trend has been reverted, and so the automatic control system switches to the "hold"' state.

The foregoing description provides for the normal operation of the automatic control system. In practice, however, occasional events necessitate the addition of another state and several other state transitions. For example, the bed occupant may move while the system is in the "reduce air" state. This movement may cause one or more indicators to increase (where otherwise they would have continued to decrease), incorrectly causing the system to switch into the "hold" state. For this reason, it is preferable to set a limit on the length of time that the system remains in the "hold" state. When the time has elapsed, the system switches to "check optimum" state. It is preferable to make the time limit variable - the first instance that the "hold" state is entered since the bed is occupied, the time limit may be quite short, perhaps only a few seconds. When the system subsequently enters a "hold" state (after cycling through the "check optimum" and "reduce" air states), if the air pressure is similar to the last air pressure while in "hold" state, then the time limit may be set to a larger value, perhaps several minutes to hours in length.

Occasionally, the patient may move in a manner that causes the air bladder to bottom-out. For example, a patient who is initially lying down may sit up. Although the air pressure in the bladder was sufficient to support the occupant while lying, it is likely that it is not sufficient to support the occupant in a seated position, causing the air bladder to collapse and bottoming-out to occur. In general, when bottoming-out occurs, the indicators will steeply increase. The present system discriminates between the slight increase in indicators indicative of the bottoming-out trend starting to occur and the steep sudden increase in several indicators that is indicative of an actual bottom-out event. If, in any of the "reduce air", "hold", "check optimum", or "increase air" states, the more than two of the indicators increase, it it indicates that a bottom-out event has occurred, and the automatic control system switches to "bottom-out recovery" state. In "bottom-out recovery" state, the automatic control system instructs the air regulator to increase the air pressure by some increment. After a period of time, the indicators are computed. If the indicators are not consistent with each other (i.e. some are increasing, others decreasing) it it indicates that the system is still bottomed-out, and the automatic control system remains in "bottom-out recovery" state, and increments the air pressure set-point once again. If all the indicators are increasing, it indicates that the system has recovered from bottoming-out, and furthermore, the bottoming-out trend has reverted, and the automatic control system switches to "reduce air" state. If all of the indicators are decreasing, it indicates that the system has recovered from bottoming-out, but that the bottoming-out trend has not yet been reverted, and the automatic control system switches to "increase air" state.

The present invention includes features to control the stability of the control system by limiting the possible state transitions. For example, only one direct transition is permitted between "Reduce Air" to "Increase Air" states in order to avoid unstable behaviour (as evidenced by the system oscillating between those states). The permitted transition occurs only if maximum pressure is detected.

A first embodiment of the pressure-relief support surface of the present invention includes a cover and a plurality of layers of a three-dimensional material located within an interior region of the cover.

The three-dimensional material is an air permeable network of fibers that has resilient, spring-like qualities, and allows for internal air circulation, for example, to provide cooling to aid in wound healing and minimize patient perspiration. The circulated air could be air that is above, at, or below ambient temperature in order to warm the patient if the patient is cool and vice versa, or achieve other desired therapeutic effects.

The three-dimensional material also has low-friction characteristics; that is, it is able to move or slide along with the movement of the patient on the support surface to reduce shear forces.

In certain embodiments, the three-dimensional material is a collapsible, slidable or lockable material. In general, the three-dimensional material is made of a woven, knitted, or non-woven fabric which comprises thermoplastic fibers or monofilaments. In one embodiment, the three-dimensional material is a breathable monofilament polyester mesh fabric that is formed into various three-dimensional patterns after weaving such as is manufactured by Freudenberg & Co. of Weinheim, Germany.

In other embodiments, a three-dimensional knit material, such as is manufactured by Tytex Group (Tytex Inc. of Rhode Island, U.S.A.) is used in place of or in addition to the SpaceNet or other three-dimensional material.

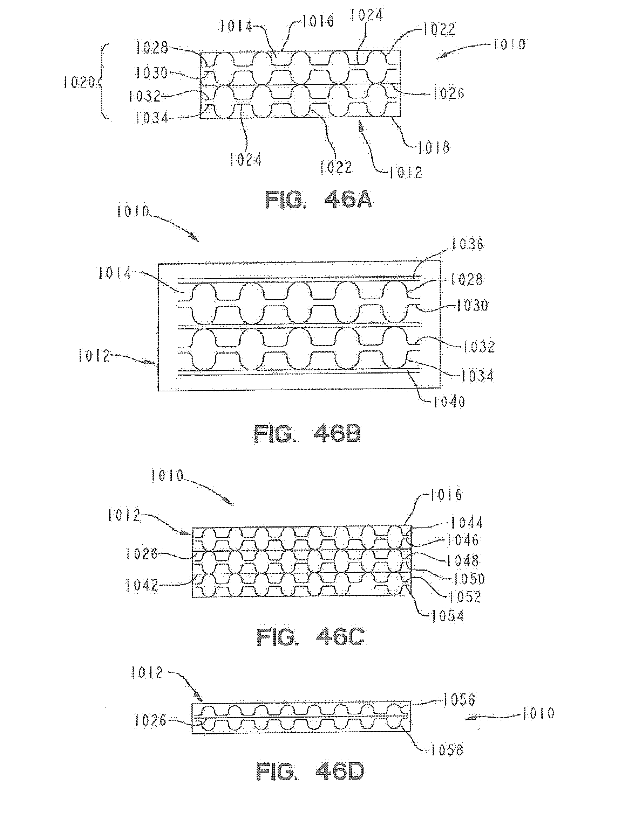

Figs. 46a-46f illustrate alternative embodiments of a support surface including a three-dimensional material located within an interior region of a cover. As particularly shown in Figs. 46a-46f, the illustrated three-dimensional material generally includes a plurality of alternating dome- or semicircular-shaped projections and depressions, or peaks and troughs.

Specific dimensions of these peaks and troughs may be mentioned in connection with particular embodiments discussed below, but it is understood that these dimensions are not so limited. Any type of three dimensional material, with peaks and troughs of any size may be used. In certain embodiments, these dimensions are adjusted to, for example, achieve particular support characteristics.

Fig. 46a is a side view of a first embodiment of a support surface 1010 including the three-dimensional material located inside a cover 1012. As shown in Fig. 46a, the cover 1012 defines an interior region 1014, which contains a plurality of layers of three-dimensional material 1020. As illustrated in Fig. 46a, there are four individual layers or strips 1028, 1030, 1032, 1034 of the three-dimensional material provided within the interior region 1014 of the cover 1012. Each individual layer of three-dimensional material includes a plurality of peaks or substantially dome-shaped projections 1022 and troughs or depressions 1024.

As illustrated in Fig. 46a, there are two layers 1028, 1030 of three-dimensional material stacked "back-to-back", with the dome-shaped projections or peaks facing in opposite directions, located above a separator material 1026, and two layers 1032, 1034 of the three-dimensional material stacked or positioned back-to-back below the separator material 1026. The dome-shaped projections or peaks 1022 and depressions or troughs 1024, respectively, are substantially aligned. The separator material 1026 is comprised of the same material used for the cover 1012, or another suitable divider material. In the illustrated embodiments, the separator material 1026 is breathable or air permeable. Alternatively or in addition, the separator material 1026 provides support for the layers 1028, 1030. In alternative embodiments, no separator material 1026 is used.

The cover 1012 has a top surface 1016 and a bottom surface 1018. A first sublayer 1028 of the three-dimensional material has dome-shaped projections 1022 projecting upwardly and located adjacent the top surface 1016 of the cover within the interior region 1014. A second sublayer 1030 of the three-dimensional material has dome-shaped projections 1022 facing downwardly and located adjacent the separator material 1026. A third sublayer 1032 of the three-dimensional material has dome-shaped projections 1022 facing upwardly toward and adjacent to the separator material 1026. A fourth sublayer 1034 of the three-dimensional material has dome-shaped projections 1022 projecting downwardly toward the bottom surface 1018 of the cover 1012.

Fig. 46b illustrates an alternative embodiment of the support surface 1010, which is similar to the embodiment shown in Fig. 46a, except that within the interior region 1014 of the cover 1012, there is located three layers of a three-dimensional spacer material 1036, 1038, 1040. The first layer of spacer material 1036 is located above the first sublayer 1028 of three-dimensional fabric. The second layer 1038 of three-dimensional spacer material is located between the second and third sublayers 1030,1032 of three-dimensional material. The third layer 1040 of three-dimensional spacer fabric is located below or underneath the fourth sublayer 1034 of three-dimensional material.

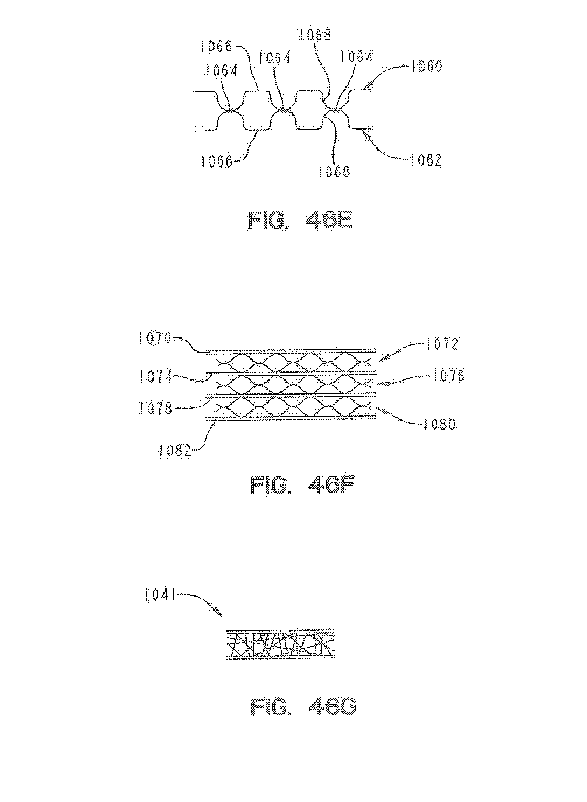

The layers of three-dimensional spacer material 1036, 1038, 1040 are made of an air permeable spacer fabric 1041. In general, the three-dimensional spacer fabric is a lightweight material that also has a cushioning effect and is breathable and able to transfer moisture. In the illustrated embodiments, the spacer fabric is a three-dimensional knit spacer fabric manufactured by Tytex Group. In one embodiment, the three-dimensional spacer fabric is latex-free. Fig. 46g is a side view of one form of spacer fabric 1041.

Fig. 46c shows another alternative embodiment of the support surface 1010, which is similar to the embodiment shown in Fig. 46a, except that it it induces a second layer of a separator material 1042 and two additional individual layers 1052, 1054 of the three-dimensional material. As shown in Fig. 46c, first and second sublayers 1044, 1046 of the three-dimensional material are located above the first separator material 1026. Second and third sublayers 1048, 1050 of the three-dimensional material are located between the first separator material 1026 and the second separator material 1042. The third and fourth individual layers 1052, 1054 of three-dimensional material are located between the second separator material 1042 and the bottom surface 1018 of the cover 1012.

The layers of separator material 1026, 1042 are comprised of the same material as is used for the cover 1012, a three-dimensional spacer fabric as described above, or other similar suitable material.

Fig. 46d shows yet another alternative embodiment of the support surface 1010. In Fig. 46d, a first individual layer 1056 of three-dimensional material is separated by a separator material 1026 from a second individual layer 1058 of three-dimensional material, within the cover 1012, so that there is only one individual layer of three-dimensional material on either side of the separator material 1026. The peaks or dome-shaped projections and troughs or depressions of the layers 1056 and 1058 are substantially aligned as discussed above.

Fig. 46e shows a side view of two back-to-back individual layers of three dimensional material 1060, 1062 which are positioned so that the peaks or dome-shaped projections 1066 and troughs or depressions 1068 are aligned directly above or below each other. The material located between the peaks and depressions 1066, 1068 of the layers 1060, 1062 is welded together at points 1064. Welding, joining, or otherwise fastening the material together at points 1064 maintains the back-to-back alignment of the peaks and depressions 1066, 1068. It is understood that in any of the illustrated embodiments, the material may be welded as shown in Fig. 46e.

Fig. 46f shows still another embodiment of the three-dimensional material located within the cover 1012 of the support surface 1010. In the embodiment of Fig. 46f, there are four separator layers 1070, 1074, 1078, 1082 are each made off the three-dimensional spacer fabric discussed above. Between the first and second layers 1070, 1074 of the spacer fabric is a pair of layers 1072 of the three-dimensional material aligned back-to-back as discussed above. Located between the second and third layers 1074, 1078 of spacer its a pair of individual layers 1076 of three-dimensional material aligned back-to-back as discussed above. Between the and fourth layers 1078, 1082 of spacer fabric is another layer 1080 comprised of two back-to-back layers of three-dimensional material, In certain embodiments, the individual layers of three-dimensional material that make up each sublayer 1072, 1076, 1080 are held together by welding, plastic ties or other suitable fasteners.

In certain particular embodiments, the height of the projections and depressions of the three-dimensional material illustrated in Figs. 46a-46f is about 3.1mm. Also in certain embodiments, the height of three-dimensional spacer fabric 1041 illustrated in Fig. 46g is about 0.2 inches. Thus, in these embodiments, when two projections of three-dimensional material are positioned back-to-back, and a spacer material is used, the total height from the top of the upper projection to the bottom of the lower projection equals about 0.44 inches. In other embodiments, the three-dimensional material and spacer fabric have different dimensions and thus the layers or combination of layers have different heights.

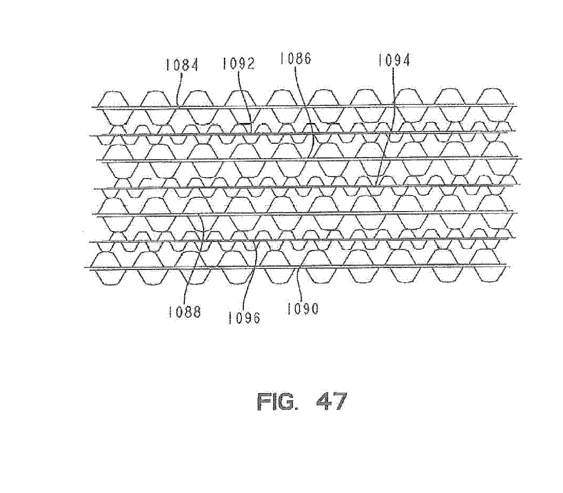

Fig. 47 shows yet another embodiment of the three-dimensional material located within the cover 1012 of the support surface 1010. In the embodiment of Fig. 47, there are four layers 1084, 1086, 1088 and 1090 of a first type or style of three-dimensional material, and three layers 1092, 1094, 1096 of a second type or style off three-dimensional material. The layers 1092, 1094, 1096 have smaller projections and depressions than the layers 1084, 1086, 1088, 1090. In other words, the projections and depressions of layers 1092, 1094, 1096 each have a diameter and/or height that is smaller the diameter and/or height of the projections and depressions of layers 1084, 1086, 1088, 1090.

All of the layers 1084, 1086, 1088, 1090, 1092, 1094, 1096 include two individual layers of three-dimensional material positioned back-to-back, however, the projections and depressions of layers 1092, 1094, 1096 are not substantially aligned as they are in the layers 1084, 1086, 1088, 1090.

In alternative embodiments, a spacer fabric is provided in between one or more of the layers or sublayers, It is understood that, in alternative embodiments of the support surface 1010, there are varying numbers of layers and/or sublayers of three-dimensional material and spacer fabric. For example, in general, the number of layers or sublayers is between 1 and 20. In one embodiment the number of layers is 1012.

In the illustrated embodiments, the cover 1012. which defines the interior region within which the three-dimensional material is positioned to form a support surface, is made of a stretchy, breathable material such as Lycra®. It is understood that any of the illustrated embodiments of Figs. 4a-4f may be inserted into the interior region 1014 of the cover 1012 to form the support surface 1010, In alternative embodiments, any of the configurations shown in Fig. 46a-46f constitute one layer and multiple such layers are inserted within the interior region 1014 of the cover 1012. In certain embodiments, the support surface 1010 constitutes one layer, for example, as a "topper' or coverlet, positioned above, below, or in between one or more other layers of patient support 10. In still other embodiments, additional layers of one or more other support materials, such as foam and/or air bladders, are also included within the interior region of the cover.

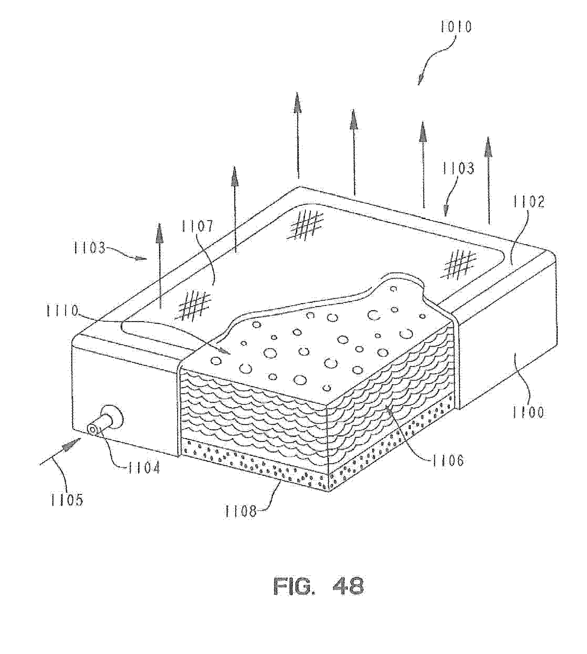

For example, in one embodiment, the support surface 1010 includes a three-dimensional material and a foam base. One such alternative embodiment is shown in Fig. 48. In the embodiment of Fig. 48, a cover 1100 includes a top surface 1102 and an air inlet 1104. At least a portion 1107 of the top surface 1102 is air permeable and permits air flow in the direction of arrows 1103. The air inlet 1104 is coupled to an air supply (not shown) so that air flows in the direction of arrow 1105 into the interior region 1110 of the cover 1100 through the air inlet 1104. Because at least a portion 1107 of the top surface 1102 permits air flow, the air that flows into the interior region 1110 flows through the interior region 1110 and then upwardly out through the top surface 1102.

The air circulated through the support surface is generally at ambient temperature. It is within the scope of the invention that various temperatures of air above and below the ambient temperature could be circulated. In alternative embodiments, the air is heated or cooled prior to circulation. In such embodiments, the air temperature is controlled by the patient or caregiver, or is automatically controlled in response to a measurement of the patient's temperature or surface temperature of the patient support. In still other embodiments, top surface 1102 is vapor and moisture permeable but air impermeable. The air does not exit top surface 1102 but exits through an opening or slit (not shown) in a head end 1103 of support surface 1010, In yet another embodiment, fluid is circulated through the support surface. The fluid could include water, refrigerant, gel, or any other suitable fluid for heating and cooling a patient.

A plurality of layers of three-dimensional material 1106 and a foam base 1108 are located in the interior region 1110 of the cover 1100. The plurality of layers off three-dimensional material 1106 may be configured in any of the ways shown in Figs. 46a-46f, 47, and 51-53a, b. In the illustrated embodiments, the three-dimensional material 1106 is of the type commonly known as Spacenet. However, it is understood that other suitable three-dimensional networked fiber materials may be used.

The foam base 1108 is positioned underneath the plurality of layers of three-dimensional material 1106 within the interior region 1110 of the covet 1100. In the illustrated embodiment, the base 1108 is constructed of reticulated foam. As illustrated, the foam base 1108 has a thickness of about 1 inch. However, it is understood that other suitable thicknesses and types of foam may be used. In alternative embodiments, foam base 1108 is not included within cover 1100 or not used at all, The embodiment of the support surface 1010 shown in Fig. 48 is thought to be particularly useful to support the area underneath a patient's heels while that patient is lying on a hospital bed, for example. The air flow through the top surface 1102 provides a cooling effect, and the resilient qualities of the three-dimensional material 1106 are configured to reduce the interface pressure between the patient's heels and the top surface 1102 of the cover 1100.

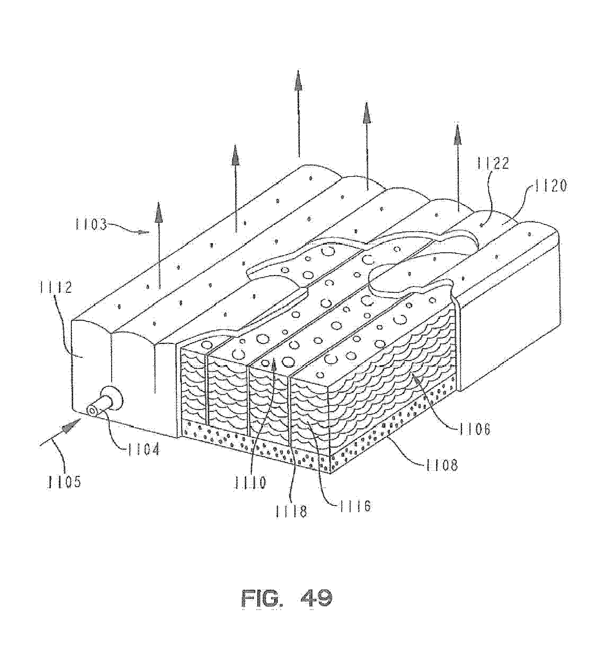

The embodiment of the support surface 1110 that is shown in Fig. 49 is similar to the embodiment of Fig. 48 except that the stack of three-dimensional layers 1106 within the interior region 1110 is divided into a plurality of columns or log-shaped cells 1116. The column 1116 are separated by channels 1118 which additionally allow air flow between the columns 1116 of three-dimensional material upwardly through the top surface 1120 of the cover 1112.

A top surface 1120 of the cover 1112 includes a plurality of pleats, valleys, indentations, or creases 1114 which generally correspond to the location of the channels 1118 within the interior region 1110. The top surface 1120 of the cover 1112 also includes a plurality of apertures 1122 which allow for air flow through the top surface 1120.

The columns 1116 of the three-dimensional material 1106 allow the three-dimensional material to move more freely in response to movement of a patient positioned on the support surface. Each individual column 1116 is movable independently of the others.

The rate of flow of the air into the interior region 1110 of the cover 1112 through the inlet 1104 can be adjusted in order to remove moisture from the interior region 1110 or from the top surface 1120 and have a drying effect on the skin of a patient or portion of a patient's body that is adjacent to the top surface 1120. Also, the rate of air flow through the inlet 1104 is adjustable. For example, it can be increased to partially or fully inflate the interior region 1110 to make the top surface 1120 firmer as may be desired, for example, for ease of transfer of the support surface or to support the patient's weight.

Still other embodiments of the support surface 1110 include a layer of three-dimensional material in combination with one or more inflatable cushions or bladders.

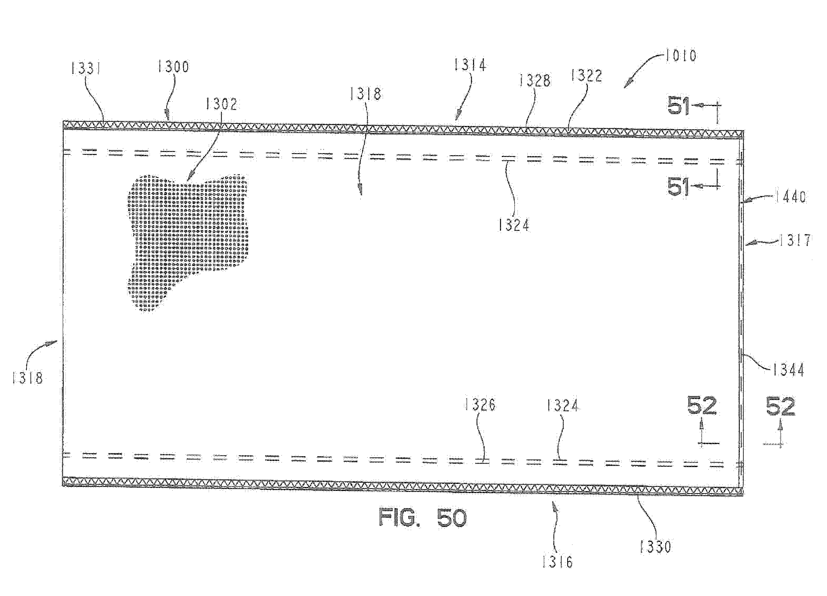

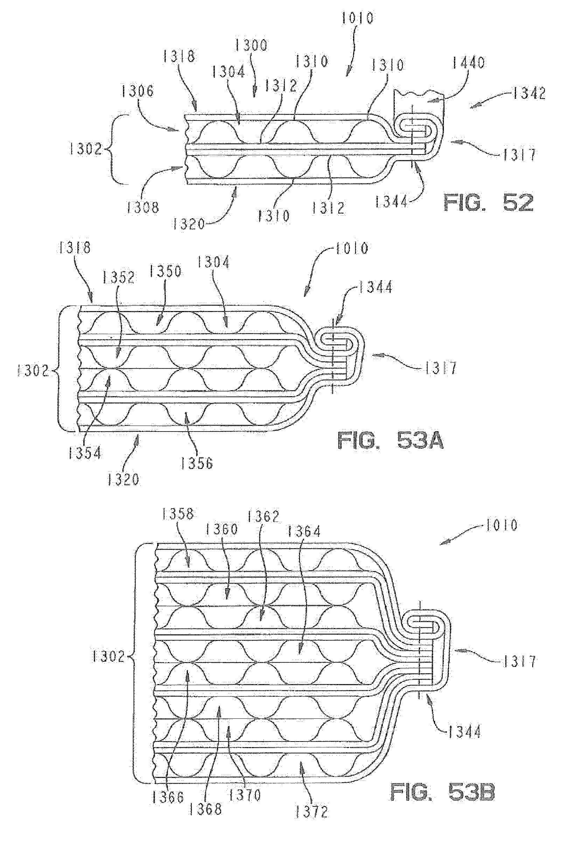

Figs. 50-52 show yet another embodiment of support surface 1010. Support surface 1010 includes a cover 1300 and a plurality of layers of three dimensional material 1302. Cover 1300 defines an interior region 1304, which contains the plurality of layers of three-dimensional material 1302. As illustrated in Figs. 52 and 52, there are two individual layers or strips 1306, 1308 of the three-dimensional material provided within the interior region 1304 of the cover 1300. Each individual layer of three-dimensional material includes a plurality of peaks or substantially dome-shaped projections 1310 and troughs or depressions 1312.

Cover 1300 includes a first longitudinal side 1314, a second longitudinal side 1316, a head end 1315, a foot end 1317, an upper cover 1318, and a lower cover 1320. A loop fastener 1322 is provided allow first and second longitudinal sides 1314, 1316. Loop faster 1322 matches to a hook fastener (not shown) located on an interior surface of a patient support cover (not shown), The hook fastener and loop fastener 1322 hold cover 1300 in place within the patient support cover.

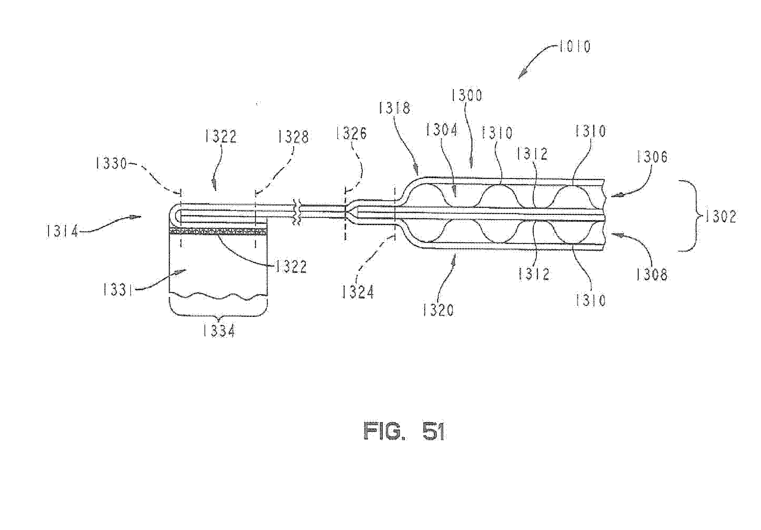

A cutaway along longitudinal side 1314 is illustrated in Fig. 51. There are two layers 1306, 1308 of three-dimensional material stacked "back-to-back", with the dome-shaped projections or peaks 1310 facing in opposite directions. The dome-shaped projections or peaks 1310 and depressions or troughs 1312, respectively, are substantially aligned.

As shown in Fig. 51, upper cover 1318 and lower cover 1320 extend beyond the two layers 1306, 1308. Upper cover 1318 and lower cover 1320 are stitched with a convention stitch at a first stitch location 1324, a second stitch location 1326, a third stitch location 1328, and a forth stitch location 1330. First stitch location is near layers 1306, 1308 and used to hold layers 1306, 1307 within cover 1300. Second stitch location 1326 is provided to reinforce first stitch location 1324. Upper and lower covers 1 31 1320 define a folded region 1331 near an end 1332 of upper cover 1318 and lower cover 1320. Stitching through folded region 1331 occurs art third and fourth stitch locations 1328, 1330. Additionally, a hem 1334 covers the entire folded region 1331. Hoop fastener 1322 is held in place by hem 1334, In alternative embodiments, upper cover 1318 and lower cover 1320 are RF Welded at the stitch and hem locations.

A cutaway along foot end 1317 is illustrated in Fig. 52. Upper and lower covers 1318, 1320 define a folded region 1340 near an end 1342 of upper and lower covers 1318, 1320. Stitching through folded region 1340 occurs at fifth stitch location 1344. A stitch or hem goes through folded region 1340. Folded region 1340 includes a portion of layers 1306, 1308 and a portion of upper and lower covers 1318, 1320.

Figs. 53A and 53B show alternative embodiments of support surface 1010 that are similar to those in Figs. 50-52. Fig. 53A shows four individual layers or strips 1350, 1352, 1354, 1356 of the three-dimensional material provided within the interior region 1304 of the cover 1300. Fig. 53B shows eight individual layers or strips 1358, 1360, 1362, 1364, 1366, 1368, 1370, 1372 of the three-dimensional material provided within the interior region 1304 of the cover 1300. In alternative embodiments, any number of layers of three-dimensional material may be used. Layers of different thickness and support characteristics could also be used. Additionally, a layer of material similar to that of the cover could be provide between each layer of three-dimensional material or between groups of layers of three-dimensional material.

As discussed above, the three-dimensional material used in certain embodiments of the support surface 1010 is generally enclosed in a cover. In embodiments of the support surface 1010 that include more than one layer of support (i.e., three-dimensional material and air bladders), an outer cover or ticking is used to enclose all of the internal layers of the support surface within an interior region.

The outer covering or ticking may be provided in addition to or in place of the cover surrounding the three-dimensional material, described above. Typically, a zipper or other suitable fastener is provided to couple two halves of the outer cover together around the support surface layers.

In general, the outer cover or ticking is made of a moisture resistant material, such as plastic or a plastic-coated material. In one particular embodiment, a urethane-coated fabric is used.

In certain embodiments, all or a portion of the outer ticking is made of a low air loss plastic or plastic-coated material, or is otherwise breathable. Alternatively or in addition, the outer ticking may be coated with a low friction material such as Teflon® to reduce sheer between the patient and the support surface. Also, the outer ticking or portions thereof may be treated with chemicals, ozone or ions so that it is bacteria resistant. Further, all or portions of the outer ticking surface may be treated or otherwise designed to resist staining, for example, using a patterned tick.

The outer ticking is generally designed to prevent fluid ingress through the use of sealed ticking or wicking channels. Also, in certain embodiments the outer ticking is designed to be disposable or replaceable.

In other embodiments, the outer cover or ticking is made of a moisture and vapor permeable but air impermeable layer. These materials are typically covered with either a Teflon® coating or a Urethane coating.

These features of the outer ticking are designed primarily to minimize the amount of maintenance required to properly care for and maintain the condition of the outer ticking and the support layers within.

The outer ticking is also configured to improve the user friendliness of the support surface 1010. For example, instructions for the caregiver with regard to appropriate installation and use of the support surface 1010 are applied to the top surface or other plainly visible areas of the outer ticking. For example, indications, icons, symbols, or distinct color coding schemes may be used to guide the caregiver through proper installation and use. Alignment decals and/or an outline of the proper orientation of a patient on the surface are also provided in certain embodiments.

Further clauses defining the invention are:

1. A patient support, comprising:

a cover (12) defining an interior region (14), a base (96) positioned in the interior region, a plurality of inflatable bladders (50), each bladder extending upwardly from the base along a vertical axis (101), the vertical axis being substantially perpendicular to the base, and a plurality of spaced-apart pressure sensors (136) positioned underneath the base, each pressure sensor being aligned with a bladder.

2. The patient support of clause 1, wherein the base includes a first base portion (97) and a second base portion (95), the second base portion is positioned above the first base portion, and the second base portion is coupled to the first base portion to define a plurality of fluid channels (112) interconnecting the bladders.

3. The patient support of clause 2, wherein the second base portion includes a plurality of apertures (196), each aperture defines a coupling region (99), and each upwardly extending bladder is coupled to the second base portion at one of the coupling regions.

4. The patient support of clause 1, wherein each pressure sensor is located beneath at least one bladder.

5. The patient support of clause 1, further comprising a pressure sensor enclosure (68, 70), wherein the pressure sensors are located within the pressure sensor enclosure.

6. The patient support of clause 5, wherein the enclosure is located within the interior region of the cover.

7. The patient support of clause 1, wherein each pressure sensor includes at least one light transmitter (128).

8. The patient support of clause 1, wherein each pressure sensor measures pressure applied to one or more of the bladders.

9. The patient support of clause 1, wherein each pressure sensor measures changes in intensity of light energy diffused in the pressure sensor.

10. The patient support of clause 1, further comprising at least one pressure transducer (328) coupled to the bladders, wherein the pressure transducer measures internal pressure of fluid in the bladders.

11. The patient support of clause 1, wherein the base includes a plurality of base sections, a plurality of bladders (60, 62, 64)are located in each of the base sections, and the bladders in each of the base sections are separately inflatable.

12. The patient support of clause 1, wherein the base includes a first base section (60, 62) and a second base section (64), the first base section bladders have a first height (116) extending from the base to a top portion of the first base section bladders, the second base section bladders have a second height (118) extending from the base to a top portion of the second base section bladders, and the first height is greater than the second height.

13. The patient support of clause 1, wherein each bladder has a top portion (98) and a vertical portion (100) extending between the top portion (102) and the base, and a beveled portion located in between the top portion and the vertical portion.

14. A patient support, comprising:

a cover (12) defining an interior region (14), a base (96) located in the interior region, the base having a first end (32) and a second end (34) longitudinally spaced apart from the first end, a plurality of substantially can-shaped inflatable bladders (50) coupled to the base, and a support layer (20) positioned above the substantially can-shaped inflatable bladders.

15. The patient support of clause 14, wherein the support layer includes a breathable material.

16. The patient support of clause 14, wherein the support layer includes a plurality of resilient portions.

17. The patient support of clause 14, wherein the support layer includes a plurality of projections (22) and depressions (24).

18. The patient support of clause 14, wherein the inflatable bladders have a first support characteristic, the support layer has a second support characteristic, and the first support characteristic is different than the second support characteristic.

19. The patient support of clause 14, further comprising a support layer enclosure (1012) , wherein the support layer is located within the support layer enclosure.

20. A patient support, comprising:

a cover (12) defining an interior region (14),
a first support layer (20) located in the interior region, the first support layer having a first support characteristic,
a second support layer (50) positioned underneath the first layer, the second support layer having a second support characteristic, and
a plurality of removable filler portions (80, 82) selectable to conform the patient support to one of a first deck configuration and a second deck configuration.

21. The patient support of clause 20, wherein the first deck configuration includes a step deck and the second deck configuration includes a flat deck.

22. A pressure adjustable mattress system (10), comprising:

a plurality of air bladders (50);
a plurality of force sensors (136), each of the plurality of pressure sensors subtending at least one of the plurality of air bladders (50) to sense a force transmitted through the subtended air bladder; and
a plurality of outputs (132), to transmit a signal representative of a sensed force, each of the plurality of outputs coupled to at least one of the plurality of force sensors.

23. The pressure adjustable mattress of clause 22, wherein the plurality of force sensors comprise a light responsive device disposed in a compressible medium.

24. The pressure adjustable mattress of clause 23, further comprising a converter (560) operatively coupled to each of the plurality of outputs, the converter including a digitizer to digitize the signal representative of the sensed force, and a filter, to filter the signal representative of the sensed force.

25. A method for adjusting the pressure of a pressure adjustable mattress to a pressure value, the mattress including a plurality of force sensors wherein each of the plurality of force sensors generates a force signal representative of a sensed force transmitted through the pressure adjustable mattress comprising:

ordering the force signals in a predetermined order;
calculating an indicator signal as a function of the ordered force signals;
comparing the indicator signal to the predetermined value to generate a correction signal; and
adjusting the pressure of the pressure adjustable mattress based on the correction signal.

26. The method of clause 25, wherein the ordering step comprises ordering the force signals in a predetermined order by reading the force signals in a predetermined sequence.

27. The method of clause 26, wherein the calculating step comprises calculating an indicator signal as a function of the ordered force signals by detecting a bottoming-out trend.

28. The method of clause 27, wherein the calculating step comprises calculating the indicator signal as a function of a standard deviation of the generated force signals.

29. In a pressure adjustable support, including a bladder to support a patient and having a pressure therein, a sensor subtending the bladder to sense a force transmitted through the bladder and generating a signal responsive to the sensed force, a method of comprising the steps of:

detecting a position of the patient on the bladder with the sensor;
detecting movement of the patient on the bladder with the sensor; and
adjusting the pressure within the bladder responsive to the detected position and the detected movement of the patient.

30. The method of clause 29, further comprising the step of pressurizing the bladder to an initial pressure.

31. The method of clause 30, the step of adjusting the pressure including reducing the initial pressure to a predetermined pressure.

32. The method of clause 31, the step of adjusting the pressure including reducing the predetermined pressure to a pressure determined according to the detected position of the patient and the detected movement of the patient.

33. The method of clause 32, the step of detecting the position of the patient including detecting a right side lying position and a left side lying position.

34. The method of clause 29, the step of detecting the movement of the patient step including detecting the patient in a sitting position.

35. The method of clause 34, the step of adjusting the pressure including adjusting the pressure within the bladder to a pressure sufficient for a patient in a sitting position.

36. In a pressure adjustable support, including a bladder assembly having a plurality of vertical bladders, to support a patient, a plurality of sensors, each of the plurality of sensors subtending at least one of the vertical bladders to tense a force transmitted through the vertical bladders, a method of comprising the steps of:

detecting a position of the patient on the plurality of vertical bladders with the plurality of sensors, detecting movement of the patient on the plurality of vertical bladders with the plurality of sensors; and

the pressure within the bladder responsive to the detected position and the detected movement of the patient.

37. The method of clause 36, further comprising the step of generating a plurality of signals responsive to the sensed force, each of the plurality of signals generated by one of the plurality of sensors.

38. The method of clause 37, further comprising reading each off the plurality of signals generated by each of the plurality of sensors in a predetermined order.

39. The method of clause 38, further comprising applying a mathematical function to the plurality of signals to determine an indicator signal to indicate a bladder pressure.

40. The method of clause 39, the step of reading each of the plurality of signals includes reading each of the plurality of signals oven a predetermined time period.

41. The method of clause 40, the of applying a mathematical function to each of the plurality of signals includes determining the standard deviation divided by the average.

42. In a pressure adjustable support, including a bladder assembly having a plurality of bladders to support a patient, a plurality of sensors, each of the plurality of sensors subtending at least one of the vertical bladders to sense a force transmitted through the bladders, a method comprising the steps of:

setting a time period for determining an activity level;

setting a threshold level with respect to the sensed force;

sampling the forces sensed by each of the plurality of sensors transmitted through the and generating a signal as a function of the set time period, the set threshold level, and the sampled forces.

43. The method of 42, the step of sampling the forces sensed by each of the plurality of sensors including sampling over a predetermined period of time the forces sensed by each of the plurality of sensors,

44. The method of clause 43, the step of sampling the forces sensed by each of the plurality of sensors including filtering the sampled forces to determine includes a high frequency component.

45. The method of clause 43, further comprising the step determining which of the filtered sampled forces exceed the set threshold level,

46. The method of 45, further comprising the step of determining the number of filtered sampled forces determined to cross the threshold level.

47. The method of clause 46, comprising the step of determining the amount of time the filtered sampled forces exceed the threshold level during the predetermined of time.

48. The method of 47, comprising the step of determining a sum of the total amount of the filtered sampled forces exceeding the threshold level,

49. The method of 48, further comprising the step of determining the maximum filtered sampled force.

50. The method of clause 49, further comprising the step of storing the maximum filtered sampled force.

51. The method of clause 50, further comprising the step of determining a weight for at least one of the number of filtered sampled forces exceeding the threshold level, the amount of time the filtered sampled forces exceed the threshold level during the predetermined period of time, sum of the total amount of the filtered sampled forces exceeding the threshold level, and the maximum filtered sample force,

52. The method of clause 42, wherein the bladder assembly includes a plurality of vertically oriented bladders.

53. The method of clause 52, wherein the bladder assembly includes an air chamber shared by each of the plurality of vertically oriented bladders.

54. The method of clause 42, wherein each of the plurality off sensors comprise a light responsive device disposed in a compressible medium.

55. The method of clause 54, the step of sampling the forces sensed by each of the plurality of sensors including sampling the forces in a predetermined order.

56. The method of clause 55, the step of sampling the forces in a predetermined order wherein the predetermined order includes sampling the forces consecutively from non-adjacent sensors.

57. A motion monitor device for monitoring the motion of a patient lying on a hospital bed (2), including a mattress (10) comprising:

a plurality of sensors (136) subtending the mattress; and

a user interface device (42), operatively coupled to the plurality of sensors, the user interface device including a screen (44) to display motion information and an input device to input motion parameters to determine patient motion.

58. The motion monitor device of clause 57, wherein the input devices includes a time period selector to select a time period over which the motion of the patient is determined.

59. The motion monitor device of clause 58, wherein the input device a threshold selector (828) to select a threshold for movement above which the patient is determined to be mobile.

60. The motion monitor device of clause 59, wherein the input device induces a history selector (832) to select a history of the monitored motion,

61. The monitor device of clause 59, wherein the user interface device includes an alarm to indicate when motion of a patient over time falls below the selected threshold level.

62. The motion monitor device of clause 57, further inducing a processor operatively to the user interface device and to the plurality of sensors, the processor to receive the motion parameters from the input device and to transmit information to the screen.

63. The motion monitor device of clause 62, wherein the processor generates a mobile signal responsive to movement of a patient sensed by the plurality of sensors for display on the screen.

64. The motion monitor device of clause 63, wherein the processor generates an immobile signal responsive to movement of a patient sensed by the plurality of sensors for display on the screen.

65. The motion monitor device of clause 64, wherein the processor generates a bed empty signal responsive to an empty bed sensed by the plurality of sensors for display on the screen.

66. A patient support surface, comprising:

a cover (1012) defining an interior region (1014), the cover inducing a top surface (1016) and a bottom surface (1018);
a first layer of a three-dimensional material positioned in the interior the three-dimensional material comprising a network of thermoplastic fibers, the network comprising a plurality of spaced-apart dome-shaped projections (1022), the dome-shaped projections projecting upwardly toward the top surface of the cover;
a second layer (1030) of the three-dimensional fiber material positioned in the interior region below the first layer, the dome-shaped projections off the second layer projecting downwardly away from the first layer toward the bottom surface of the cover; and
a plurality of vertical can bladders (50) positioned in the interior region below the second layer.

67. The patient support surface of clause 66, wherein the dome-shaped projections of the second layer are substantially aligned with the dome-shaped projections of the first layer.

68. The patient support surface of clause 67, further comprising a third layer (1032) substantially similar to the first layer, wherein the third layer is positioned below the second layer, and the dome-shaped projections of the third layer project upwardly toward the second layer.

69. The patient support surface of clause 68, further comprising a fourth layer (1034) substantially similar to the second layer, wherein the fourth layer is positioned below the third layer, and the dome-shaped projections of the fourth layer project downwardly toward the bottom surface of the cover.

70. The patient support surface of clause 69, further comprising a layer of three-dimensional spacer fabric (1036) located in between the second and third layers.

71. The patient support surface of clause 70, wherein the first, second, third and fourth layers and the layer of spacer fabric together comprise a sublayer, and a plurality of the sublayers are arranged one on top of the other in the interior region of the cover.

72. The patient support surface of clause 66, wherein each dome shaped projection has a top, and a bottom, and a distance from the top of a dome-shaped projection of the first layer to the bottom of a dome-shaped projection of the second layer is about 0.44 inches.

73. The patient support surface of clause 66, wherein the first and second layers comprise a sublayer (1044), and at least three sublayers (1044, 1048, 1050) are positioned one on top of the other within the interior region of the cover.

74. The patient support surface of clause 73, wherein the number of sublayers is between 1 and 20.

75. The patient support surface of clause 66, wherein the dome-shaped projections of the first and second layers are substantially aligned while projecting in opposite directions.

76. The patient support surface of clause 66, wherein the top surface of the cover is air permeable.

77. A patient support surface, comprising:

an outer cover (12) defining an interior region (14);
a support layer (1010) positioned in the interior region, the support layer including a support cover (1300), an upper section (1306), and a lower section (1308), wherein the upper and lower sections are formed from a three-dimensional material (1302) comprising a network of thermoplastic fibers; and
a plurality of vertical can bladders (50) positioned in the interior region below the support layer.

78. The patient support surface of clause 77, wherein the upper section includes a first layer (1350) of the network of thermoplastic fibers and a second layer (1352) of the network of thermoplastic fibers, the first layer of the network of thermoplastic fibers comprising a first plurality of spaced-apart dome-shaped projections (1310), the first plurality of dome-shaped projections projecting upwardly toward a top surface of the support cover, the second layer of the network of thermoplastic fibers comprising a second plurality of spaced-apart dome-shaped projections, the second plurality of dome-shaped projections projecting downwardly toward a bottom surface of the support cover.

79. The patient support surface of clause 77, wherein the lower section includes a first layer (1354) of the network of thermoplastic fibers and a second layer (1356) of the network of thermoplastic fibers, the first layer of the network of thermoplastic fibers comprising a first plurality of spaced-apart dome-shaped projections, the first plurality of dome-shaped projections projecting upwardly toward a top surface of the support cover, the second layer of the network of thermoplastic fibers comprising a second plurality of spaced-apart dome-shaped projections, the second plurality of dome-shaped projections projecting downwardly toward a bottom surface of the support cover.

80. A patient support surface, comprising:

a cover (12) defining an interior region (14),
a body (1010, 50) located in the interior region, the body inducing a plurality of inflatable zones (50), each zone including a plurality of vertical can bladders; and
a top layer (1010) positioned above the body in the interior region, the top layer including at least one layer of an air-permeable three-dimensional material (1302), the three-dimensional material comprising a network of thermoplastic fibers three-dimensional material.

81. The patient support surface of clause 80, wherein the top layer includes at least four layers (1350, 1352, 1354, 1356) of the air-permeable three-dimensional material.

82. The patient support surface of clause 80, wherein the top layer includes at least eight layers (1358, 1360, 1362, 1364, 1366, 1368, 1370, 1372) of the air-permeable three-dimensional material.

83. A patient support comprising:

a cover (1010) defining an interior region;
a first layer in the interior region, the first layer including an upper section (1306) and a lower section (1308), each of the upper and lower sections including at least one layer of an air-permeable three-dimensional material (1302), the three-dimensional material comprising a network of thermoplastic fibers; and
a second layer (50) positioned below the first layer in the interior region, the second layer including head (60), seat (62), and foot (64) sections, art least one of the head, seat, and foot sections including vertical inflatable bladders.

84. The patient support surface of clause 83, wherein the vertical inflatable bladders are each substantially cylindrical in shape.

85. The patient support surface of clause 83, wherein the upper and lower sections having first and second support characteristics, respectively, the first support characteristic being different from the second support characteristic.

## Claims

1.  A patient support surface, comprising:

    a cover (1012) defining an interior region (1014), the cover including a top surface (1016) and a bottom surface (1018);
    a first layer of a three-dimensional material positioned in the interior region, the three-dimensional material comprising a network off thermoplastic fibers, the network comprising a plurality of spaced-apart dome-shaped projections (1022), the dome-shaped projections projecting upwardly toward the top surface of the cover;
    a second layer (1030) of the three-dimensional fiber material positioned in the interior region below the first layer, the dome-shaped projections of the second layer projecting downwardly away from the first layer toward the bottom surface of the cover; and
    a plurality of vertical can bladders (50) positioned in the interior region below the second layer.

2.  The patient support surface of clam 1, wherein the dome-shaped projections of the second layer are substantially aligned with the dome-shaped projections of the first layer.

3.  The patient support surface of claim 2, further comprising a third layer (1032) substantially similar to the first layer, wherein the third layer is positioned below the second layer, and the dome-shaped projections of the third layer project upwardly toward the second layer.

4.  The patient support of claim 3, further comprising a fourth layer (1034) substantially similar to the second layer, wherein the fourth layer is positioned below the third layer, and the dome-shaped projections of the fourth layer project downwardly toward the bottom surface of the cover.

5. The patient support surface of claim 4, further comprising a layer of three-dimensional spacer fabric (1036) located in between the second and third layers.

6. The patient support surface of claim 5, wherein the first, second, third and fourth layers and the layer of spacer fabric together comprise a sublayer, and a plurality of the sublayers are arranged one on top of the other in the interior region of the cover.

7. The patient support surface of any preceding claim, wherein each dome shaped projection has a top, and a bottom, and a distance from the top of a dome-shaped projection of the first layer to the bottom of a dome-shaped projection of the second layer is about 0.44 inches.

8. The patient support surface of any preceding claim, wherein the first and second layers comprise a sublayer (1044), and at least three sublayers (1044, 1048, 1050) are positioned one on top of the other within the interior region of the cover.

9. The patient support surface of claim 8, wherein the number of sublayers is between 1 and 20.

10. The patient support surface of any preceding claim, wherein the dome-shaped projections of the first and second layers are substantially aligned while projecting in opposite directions.

11. The patient support surface of any preceding claim, wherein the top surface of the cover is air permeable.

FIG. 1

FIG. 2

FIG. 3

FIG. 4

FIG. 5

FIG. 6

EP 2 250 987 A2

FIG. 7

FIG. 9

FIG. 8

FIG. 11

FIG. 10

FIG. 12A

FIG. 12B

FIG. 12C

FIG. 13

FIG. 14

FIG. 15

FIG. 16

FIG. 17

FIG. 19

FIG. 18

FIG. 20

FIG. 21

FIG. 22

FIG. 23

FIG. 24A

FIG. 24B

FIG. 25

FIG. 26

FIG. 27

FIG. 28

FIG. 29

```
                    ┌──────────────┐
                    │    Start     │
                    └──────┬───────┘
                           ↓
          ┌──────────────────────────────┐
          │   Sample Sensor Data at       │──── 802
          │   Predetermined Rate          │
          └───────────────┬──────────────┘
                          ↓
          ┌──────────────────────────────┐
          │   Compute a Motion Metric     │──── 804
          │   as a Function of Sampled Data│
          └───────────────┬──────────────┘
                          ↓
          ┌──────────────────────────────┐
          │   Set a Time Period for        │──── 806
          │   Determining Activity Metrics │
          └───────────────┬──────────────┘
                          ↓
          ┌──────────────────────────────┐
          │   Set a First Threshold        │──── 808
          └───────────────┬──────────────┘
                          ↓
          ┌──────────────────────────────┐
          │  Count Number of Motion Metrics│──── 810
          │  Crossing Threshold During Time│
          │  Period                        │
          └───────────────┬──────────────┘
                          ↓
          ┌──────────────────────────────┐
          │  Count Number of Samples of    │──── 812
          │  Sensor Data Corresponding to  │
          │  Motion Metrics Exceeding      │
          │  Threshold During Time Period  │
          └───────────────┬──────────────┘
                          ↓
          ┌──────────────────────────────┐
          │  Sum Motion Metrics Exceeding  │──── 814
          │  Threshold During Time Period  │
          └───────────────┬──────────────┘
                          ↓
          ┌──────────────────────────────┐
          │  Store Maximum Motion Metric   │──── 816
          │  During Time Period            │
          └───────────────┬──────────────┘
                          ↓
          ┌──────────────────────────────┐
          │  Calculate Weighted Sum of     │──── 818
          │  Results From Steps            │
          │  810, 812, 814, 816            │
          └───────────────┬──────────────┘
                          ↓
          ┌──────────────────────────────┐
          │ Generate Warning Signal if     │──── 820
          │ Weighted Sum is Less than      │
          │ Second Threshold               │
          └──────────────────────────────┘
```

FIG. 30

FIG. 31

FIG. 32

EP 2 250 987 A2

EP 2 250 987 A2

FIG. 33

FIG. 34

EP 2 250 987 A2

EP 2 250 987 A2

**Motion Monitor**

824

24 22 20 18 16 14 12 10 8 6 4 2 Now ▽ High

Low

Graph View

826

| -2 day | -1 day | Today |

850    848

822

830 — Help (?)

832 — History

834 — Done ✓

**FIG. 35**

FIG. 36

EP 2 250 987 A2

Motion Monitor

Help

History

Done

Now
High

Low

4 hours ago
2

876
874
860

Bed Empty
868

Immobile
870

Mobile
872

862
864

Alert Period

OFF | 30 min | 1 hour | 1.5 hour | 2 hour

866

FIG. 37

FIG. 38

FIG. 39

FIG. 40

711 — Start at High Air Pressure

712 — Reduce the Air Pressure by a Fixed Increment

713 — Compute the Bottom-Out Indicators

714 — Advance Notice of Bottom-Out?

No

Yes

715 — Optimum Reached

FIG. 41

FIG. 42

FIG. 43

FIG. 44

FIG. 45

FIG. 46A

FIG. 46B

FIG. 46C

FIG. 46D

1066 1068 1064
1064 1064 1060

**FIG. 46E**

1066 1068 1062

1070 1072
1074 1076
1078 1080
1082

**FIG. 46F**

1041

**FIG. 46G**

FIG. 47

FIG. 48

FIG. 49

FIG. 50

FIG. 51

FIG. 52

FIG. 53A

FIG. 53B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6499167 B, Ellis
- WO 200400678A1 A
- WO 2004006768AL A
- WO 2004006768 A, Lokhorst